# EUROPEAN PATENT SPECIFICATION

(11) **EP 1 694 298 B1**
(45) Date of publication and mention of the grant of the patent: **09.06.2010**
(21) Application number: 04799314.2
(22) Date of filing: 03.11.2004
(51) Int. Cl.: A61K 9/127

(54) **METHOD FOR SELECTING CATIONIC OR ANIONIC LIPOSOMES FOR TREATMENT OF A MUCOSA MEMBRANE, AND KIT COMPRISING THE SAME**
VERFAHREN ZUR AUSWAHL VON KATIONISCHEN ODER ANIONISCHEN LIPOSOMEN ZUR BEHANDLUNG EINER SCHLEIMHAUTMEMBRAN UND DIESE ENTHALTENDES SET
PROCEDES DE SELECTION D'UN MEDICAMENT POUR UN PROTOCOLE MEDICAL, ET NECESSAIRE METTANT EN OEUVRE DE TELS PROCEDES

(30) Priority: 03.11.2003 US 516316 P
(43) Date of publication of application: 30.08.2006
(73) Proprietor: Yissum Research Development Company, of The Hebrew University of Jerusalem, 91390 Jerusalem (IL)
(72) Inventor: BARENHOLZ, Yechezkel, 93707 Jerusalem (IL); RUBINSTEIN, Abraham, 97877 Jerusalem (IL); JUBEH, Tareq, 91517 Jerusalem (IL)
(74) Representative: Duffy, Assumpta Dympna
(86) International application number: PCT/IL2004/001005
(87) International publication number: WO 2005/041932

(56) References cited:
- EP-A- 0 287 198
- EP-A- 0 287 198
- WO-A-02/089771
- WO-A1-93/14744
- WO-A1-93/14744
- CA-A1- 2 451 846
- CA-A1- 2 451 846
- US-A1- 2003 026 831
- US-A1- 2003 027 339
- JUBEH TAREQ ET AL: "The effect of liposomal charge density, dimensions, incubation time and tissue inflammation on the mucoadhesion properties of cationic liposomes in the rat colon" AAPS PHARM SCI, vol. 4, no. 4, 2002, page ABSTRACT R6152, XP002315936 ISSN: 1522-1059
- BLAU SIGAL ET AL: "Drug targeting by surface cationization" CRITICAL REVIEWS IN THERAPEUTIC DRUG CARRIER SYSTEMS, vol. 17, no. 5, 2000, pages 425-465, XP009042815 ISSN: 0743-4863
- YASUI KIYOTADA ET AL: "Positively charged liposomes containing tumor necrosis factor in solid tumors" BIOLOGICAL AND PHARMACEUTICAL BULLETIN, vol. 23, no. 3, March 2000 (2000-03), pages 318-322, XP001205127 ISSN: 0918-6158
- RENGEL RUZICA GALOVIC ET AL: "High efficiency entrapment of superoxide dismutase into mucoadhesive chitosan-coated liposomes" EUROPEAN JOURNAL OF PHARMACEUTICAL SCIENCES, vol. 15, no. 5, June 2002 (2002-06), pages 441-448, XP002315937 ISSN: 0928-0987
- JUBEH TAREQ TAHA ET AL: "Differential adhesion of normal and inflamed rat colonic mucosa by charged liposomes." PHARMACEUTICAL RESEARCH (DORDRECHT), vol. 21, no. 3, March 2004 (2004-03), pages 447-453, XP002430104 ISSN: 0724-8741
- BACZYNSKA DAGMARA ET AL: "Surface charge and the association of liposomes with colon carcinoma cells" ZEITSCHRIFT FUER NATURFORSCHUNG SECTION C JOURNAL OF BIOSCIENCES, vol. 56c, no. 9-10, September 2001 (2001-09), pages 872-877, XP009043234 ISSN: 0939-5075
- DAEMEN T ET AL: "LIPOSOMAL PHOSPHATIDYLSERINE INHIBITS TUMOR CYTOTOXICITY OF LIVER MACROPHAGES INDUCED BY MURAMYL DIPEPTIDE AND LIPOPOLYSACCHARIDE" BIOCHIMICA ET BIOPHYSICA ACTA. BIOMEMBRANES, AMSTERDAM, NL, vol. 1285, no. 2, 1996, pages 219-228, XP001204942 ISSN: 0005-2736
- KIM J-S ET AL: "IN VITRO CYTOTOXIC EFFECT OF N-(PHOSPHONACETYL)-L-ASPARTIC ACID IN LIP" ARCHIVES OF PHARMACAL RESEARCH, NATL. FISHERIES UNIVERSITY, PUSAN, KR, vol. 23, no. 2, April 2000 (2000-04), pages 167-171, XP009043235 ISSN: 0253-6269
- NAGASHIMA R: "Mechanisms of action of sucralfate." JOURNAL OF CLINICAL GASTROENTEROLOGY. 1981, vol. 3, no. Suppl 2, 1981, pages 117-127, XP009042855 ISSN: 0192-0790
- HAYDEN, S.M. ET AL.: "Binding of brush border myosin I to phospholipid vesicles" THE JOURNAL OF CELL BIOLOGY, vol. 111, August 1990 (1990-08), pages 443-451, XP002430105
- JUBEH TAREQ ET AL: "The effect of liposomal charge density, dimensions, incubation time and tissue inflammation on the mucoadhesion properties of cationic liposomes in the rat colon" AAPS PHARM SCI, vol. 4, no. 4, 2002, page ABSTRACT R6152, XP002315936 ISSN: 1522-1059
- BLAU SIGAL ET AL: "Drug targeting by surface cationization" CRITICAL REVIEWS IN THERAPEUTIC DRUG CARRIER SYSTEMS, vol. 17, no. 5, 2000, pages 425-465, XP009042815 ISSN: 0743-4863 cited in the application
- YASUI KIYOTADA ET AL: "Positively charged liposomes containing tumor necrosis factor in solid tumors" BIOLOGICAL AND PHARMACEUTICAL BULLETIN, vol. 23, no. 3, March 2000 (2000-03), pages 318-322, XP001205127 ISSN: 0918-6158
- RENGEL RUZICA GALOVIC ET AL: "High efficiency entrapment of superoxide dismutase into mucoadhesive chitosan-coated liposomes" EUROPEAN JOURNAL OF PHARMACEUTICAL SCIENCES, vol. 15, no. 5, June 2002 (2002-06), pages 441-448, XP002315937 ISSN: 0928-0987

## Description

### FIELD OF THE INVENTION

This invention relates to use of negatively charged liposomes relying on differential adhesion of normal, i.e. healthy and diseased mucosa, particularly mucosa of the gastrointestinal tract.

### LIST OF PRIOR ART

(1) Miller C.R. Biochemistry, 1998, 37:12875-12883;
(2) WO02/064109 to GW PHARMA LIMITED
(3) Sigal Blau et al. Critical Reviewed in Therapeutic Drug Carrier Systems 2000 17(5):425-466;
(4) Venkatesan, N. and Vyas, S. P. International journal of pharmaceutics. 2000, 203:169-77;
(5) Iwanaga, K. et al. J. Pharm. Sci.1999, 88:248-252;
(6) Yerushalmi, N. et al. Biochim. Biophys. Acta. 1994, 1189:13-20;
(7) Rogers, J. A. and Anderson, K. E. Crit. Rev. Ther. Drug Carrier. Syst. 1998, 15:421-480;
(8) Blau, S. et al. Crit. Rev. Ther. Drug Carr. Sys. 2000, 17:425-466;
(9) Takeuchi, H. et al. J Control Release. 2003, 86:235-42;]

### BACKGROUND OF THE INVENTION

A variety of GI diseases are confined to the intestinal mucosa, many are associated with reactive oxygen species and oxidative stress [Grisham, M., Lancet 1994, 344:859-861; Pavlick, K. P. et al. Free Rad. Biol. Med. 2002, 33(3):311-322; Van der Vliet, A. et al. Free Rad. Biol. Med. 1992, 12:499-513. For example inflammatory bowel diseases (IBD) result in an intense flux of activated neutrophils into the inflamed mucosa. The neutrophils adhere transiently, allowing the production of oxidants (superoxide and H₂O₂ mainly) only at the site of their adherence [Saveryumuttu, S. H. et al. Gut 1985, 26:378-383]. Although the mucus lining of the gastrointestinal tract possesses antioxidant properties [Grisham, M. B. et al. Am. J. Physiol. 1987, 253:G93-G96], it is exposed to a continuous oxidative damage by the efflux of oxygen reactive species. One way to reduce this stress is to introduce antioxidants capable of scavenging OH radicals that are produced by the Habber-Wiess reaction. The antiinflammatory drug 5-aminosalicylic acid was suggested to act as a scavenger of OH• radicals, and of the neutrophil-derived hypochlorous acid [Dallegri, F. et al. Gut 1990, 31(2):184-186].

Natural antioxidant enzymes such as superoxide dismutase (SOD) or catalase are also candidates for the treatment of intestinal injuries. SOD has been tested, clinically, for the treatment of Crohn's disease [Emerit, J. et al. Free Radic Res Commun 1991, 12-13(Pt 2):563-569].

Another antioxidant, Tempamin (TMN), is a stable SOD mimic, which was found to be effective in both cellular and tissue levels, against a variety of reactive oxygen species (ROS) and the insult they cause. [Samuni, A. et al. Free Radic. Res. Commun. 1991, 12-13(Pt 1):187-194], including intestinal injuries [Udassin, R. et al. Gut 1998, 42(5):623-627].

Oral administration offers a potential portal to the superficial layers, *inter alia,* of the gastrointestinal (GI) tract. Due to the disputed extent of particle uptake from the intestinal lumen into the bloodstream, the most attractive use of particulate drug carriers is topical (local) drug treatment of intestinal diseases. A typical therapeutic opportunity is ulcerative colitis, an inflammatory disease confined to the epithelium of the large intestine. Experience with drugs such as salicylates (5-aminosalicylic acid) [Christensen L.A. Dan. Med. Bull., 2000 46:20-42] and steroids (budesonide) [Rutgeerts P. Mediators, Inflamm., 1998 7:137-140] have justified local therapy in the colon.

ROS production has also been associated with tumor recurrence. SOD and catalase have been demonstrated in an animal model to dramatically reduce tumor development in a peritoneal wound site context. SOD was demonstrated to reduce tumor recurrence by 50%, while catalase by 40%. Therefore, local release of the antioxidants onto the wound site will reduce ROS production, promote wound healing and consequently inhibit tumor recurrence after cancerous polyps removal (M. E. van Rossen, et al. Cancer Res 2000, 60:5625-5629).

Attachment of cationized enzymes to the colonic mucosa has already been experiences successfully [Blau, B. et al. Pharm. Res., 2000, 9:1077-1084], however, a possible drawback of this method (which differs essentially from liposomal entrapment) is a partial loss of activity of the enzymes resulting from the chemical charge-modification and potential immunogenicity.

Lipid assemblies, such as microemulsions [Dunn, C.J. et al. Drugs, 2001 61:1957-2016] and oral liposomes [Rogers J.A. and Anderson K.E Crit. Rev. Ther. Drug Carrier Syst., 1998 15:421-480; Lian T. and Ho R.J. J. Pharm. Sci., 2001 90:667-680] have been examined as potential drug carriers, primarily in the context of increasing intestinal absorption of lipophilic drugs [Gershanik T. and Benita S Eur. J. Pharm. Biopharm., 2000 50:179-188].

Mucosal targeting by liposomes can be achieved by manipulating their surface properties. Surface polymerization and polymer-coated liposomes have been mentioned in the context of increased stability of orally administered liposomes [Venkatesan N. and Vyas S.P. Int. J. Pharm., 2000 203:169-177; Iwanaga K. et al. J. Pharm. Sci.1999, 88:248-252]. Liposomal targeting has been tested using covalently anchored collagen [Yerushalmi N. and Margalit R. Biochim. Biophys. Acta, 1994, 1189:13-20], lectins [Lehr M J. Control. Rel. 2000, 65:19-29], or carbohydrates [Rogers J.A. and Anderson K.E. 1998, ibid.]. Surface charge modification has been suggested as a common means of liposome localization to cells and a variety of body organs [Miller C.R. Biochemistry, 1998, 37:12875-12883; Cristiano R.J. Frontiers Biosci., 1998 3:D1161-D1170]. Both cationic and anionic liposomes have been tested as targetable delivery systems, where the electrostatic interaction with the surface of cells was the leading cause for liposome attachment to the cell membrane, thus leading to their internalization [Miller C.R. et al. **1998** ibid.; Blau S. et al. Crit. Rev. Ther. Drug. Sys., 2000, 17:425-466*].*

### SUMMARY OF THE INVENTION

The invention is based on the surprising finding that there is a differential attachment of charged liposomes to either normal or inflamed intestinal mucosa. Specifically, it has been established that cationic liposomes adhere to a healthy mucosa better than neutral or anionic liposome, while, anionic liposome adherence to an inflamed mucosa was better than that of either neutral or cationic liposomes. Further, it was established that adherence directly correlated with charge density.

Thus the present invention provides the use of negatively charged liposomes loaded with an active ingredient that is not covalently bound to said liposomes in the manufacture of a medicament for treatment of a disease or disorder associated with inflammation of the mucosa of the gastrointestinal (GI) tract selected from ulcerative colitis, Crohn's disease and colitis.

As used herein the term *"**medical procedure**"* denotes treatment, i.e. for the purpose of curing, of a condition or prevention of a condition from developing in the tissues forming the mucosa. For the purpose of curing, the term *"**treatment**"* includes, without being limited thereto, administering of an amount of a medicament comprising an active ingredient and positively charged (cationic) lipid assemblies, the amount being effective to ameliorate undesired symptoms associated with the condition, to slow down progression of the condition or delay the onset of the progressive stage, to slow down deterioration of such symptoms, to enhance onset of a remission period of the condition, if existing, to delay onset of a progressive stage, to improve survival rate or more rapid recovery from the condition, to lessen the severity or to cure the condition etc.

The term *"**prevention**"* includes, without being limited thereto, administering of an amount of a medicament comprising an active ingredient and negatively charged (anionic) lipid assemblies to prevent the condition from occurring in the layers confined in the mucosa, to prevent irreversible damage caused by the condition, to prevent the manifestation of symptoms associated with the condition before they occur, to inhibit the progression of the condition etc.

The ***medical procedure*** according to the invention may also include a combination of ***treatment*** and ***prevention*** as defmed herein. To this end, the medical procedure involves providing the subject in need of the medical procedure a combined treatment, i.e. a combination of positively charged lipid assemblies and negatively charged lipid assemblies (administered either together or separately) loaded with either the same or different active ingredients. The medicaments, i.e. that comprising the positively charged lipids (for prevention) and that comprising the negatively charged lipids (for curing) may be administered to the subject in need simultaneously or within a predefined time interval, as prescribed by the physician, based on medical considerations known to those versed in the art.

The term *"**mucosa**"* as used herein denotes the moist tissue lining body cavities (such as alimentary canal, nose, lungs, vagina), secretes mucous and covered with epithelium. Histologically, the intestinal mucosa is divided into three layers: epithelial lining, lamina propria (support), muscularis mucosa (smooth muscle layer). It is supported by the submucosa (a loose collagenous tissue contains blood vessels, lymphatics, & nerves) and the muscularis propria (smooth muscle inner circular layer, outer longitudinal layer).

In the context of the present invention mucosa membrane preferably refers to the mucosa lining the alimentary passages and cavities, i.e. the mucosa within the gastrointestinal tract.

The term *"**disease or disorder**"* as used herein denotes any condition that impairs the normal function the mucosa, preferably the gastrointestinal mucosa. The disease may be characterized by periods of varying disease activity, i.e. quiescent, intermediate, and acute (active) phases. Depending on the phase symptoms can range from none to mild and somewhat tolerable, to severe and requiring hospitalization for treatment. Non-limiting examples include conditions resulting from inflammation, exposure of the mucosa to short-term or long-term oxidative stress, motility disorders (for example in irritable bowel syndrome) and malignant processes such as colon cancer or familial adenomatous polyposis (FAP).

As used herein, the term *"**lipid assemblies**,"* denotes an organized collection of lipids forming *inter alia,* micelles and liposomes. It is essential that the lipid assemblies of the invention be stable lipid assemblies. The term *"**stable lipid assemblies**,"* refer to the stability during storage, as well as the stability after administration to the subject in need thereof. In terms of storage, stability includes chemical as well as physical stability under storage conditions (2-8°C), and in biological fluids (37°C) for at least six months. Further, it denotes that during storage the integrity and composition of the lipid assembly is substantially unaltered and if already loaded with the active ingredient, the later has a low leakage or desorption rate from the lipid assembly. To this end, the lipid assembly may be combined with stabilizers. Non-limiting examples of stabilizers include cholesterol and similar membrane active sterols, lipopolymers such as PEGylated lipids.

As used herein, the term *"**charged lipid assemblies**"* denotes assemblies having a net positive or negative charge. Accordingly, while the assembly may be composed of a combination of positively charged (cationic) and negatively charged (anionic) components, a positively charged lipid assembly is such that its *net* charge is positive, and a negatively charged lipid assembly is such that its *net* charge is negative.

As used herein, the term *"**loaded with an active ingredient**"* denotes any type of association between the active ingredient and the assembly, including electrostatic interaction, or when the assembly forms micelles and/or vesiculate (e.g. liposomes), the loading encompass encapsulation of the active ingredient within the vesicle, entrapment of the active ingredient (in whole or in part) within the lipid layer of the vesicle (insertion), electrostatic adsorption to the surface of the micelles or the vesicles or any combination of the above. Notwithstanding the above, the term "*loaded with an active ingredient*" does not include covalent binding between the lipid carrier and the active ingredient.

Loading of the active ingredient within the vesicle or liposome may be achieved by any known method of encapsulation available, including, passive as well as active (remote) loading [Haran et al. **1993** *ibid.;* Barenholz Y. J liposome Res. 13:1-8, 2003, U.S. patent Nos. 5,136,771 and 5,939,096].

As used herein, the term *"**topical treatment**"* denotes focused treatment at a limited region (preferentially where a disease exists) from the lumen aspect of the intestine (non-systemically) via the epitheliun. Accordingly, a therapeutic effect may be achieved at the various layers of the mucosal tissues, including the submucosa, muscularis mucosa and the muscle layers of the mucosa. It should be noted that by the use of charged lipid assemblies as the drug carrier, a reversible docketing is achieved, i.e. after a while the charged lipid assembly is detached from the epithelium tissue. Thus, by the use of charges lipid assemblies according to the invention, the risk of undesired side effects is reduced.

### BRIEF DESCRIPTION OF THE DRAWINGS

In order to understand the invention and to see how it may be carried out in practice, a preferred embodiment will now be described, by way of non-limiting example only, with reference to the accompanying drawings, in which:

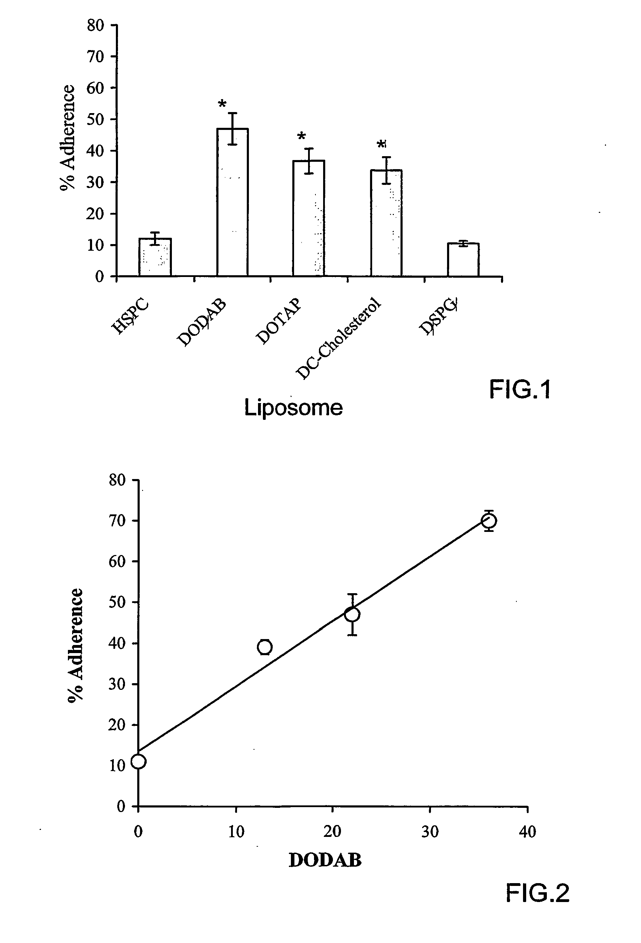

**Fig. 1** is a bar graph showing the % adherence (% adsorption out of initial amount) of three types of cationic (DODAB, DOTAP, and DC-Chol); neutral (HSPC); and anionic (DSPG) liposomes (800±50 nm) to the healthy epithelium of the rat colon after 75 min of incubation in colon sacs (* denotes *p* < 0.001 when compared to HSPC-containing liposomes). Ratio of all charged lipids was 22 mol% and the averages of six different studies ± SEM are shown.
**Fig. 2** is a graph showing the effect of increasing amounts of DODAB (expressed in mol% of total lipids in the cationic liposomes) on the percent of tissue adherence (% of initial amounts) of cationic liposomes (800±50 nm) to the healthy epithelium of the rat colon as measured after 75 min of incubation in colon sacs. The averages of six different studies ± SEM are shown.

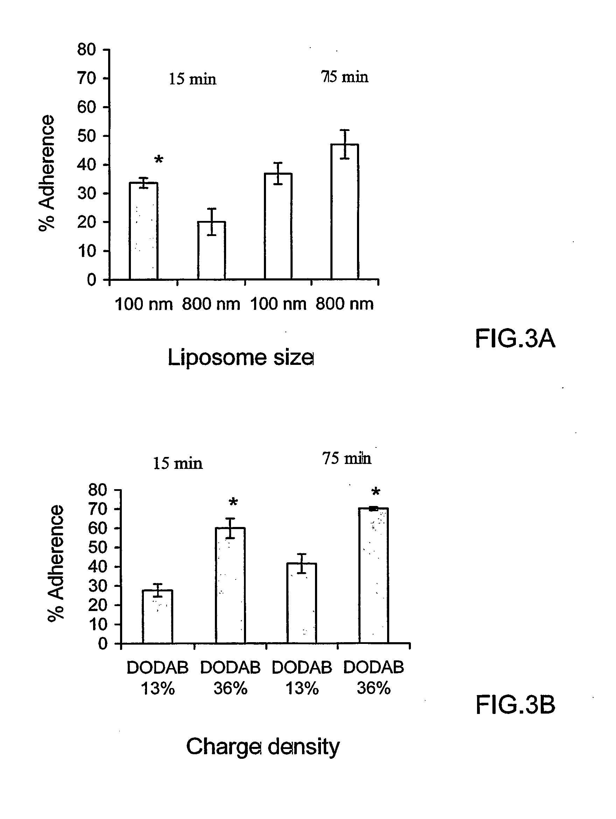

**Figs. 3A-3B** are bar graphs showing the effect of incubation time (15 or 75 minutes) and liposome size or charge density on the adherence of cationic liposomes. **Fig. 3A** shows the effect of incubation time and liposome's size on the adherence of cationic liposomes (DODAB, 22 mol% of total lipids) to the healthy epithelium of the rat colon as measured in colon sacs; **Fig. 3B** shows the effect of incubation time and charge density on the adherence of cationic liposomes (DODAB, 13 or 36 mol% of total lipids, 800±50 nm) to the healthy epithelium of the rat colon as measured in colon sacs. Shown are the averages of six different studies ± SEM. * denotes *p* < 0.005 when compared to liposomes containing 13 mol% DODAB.

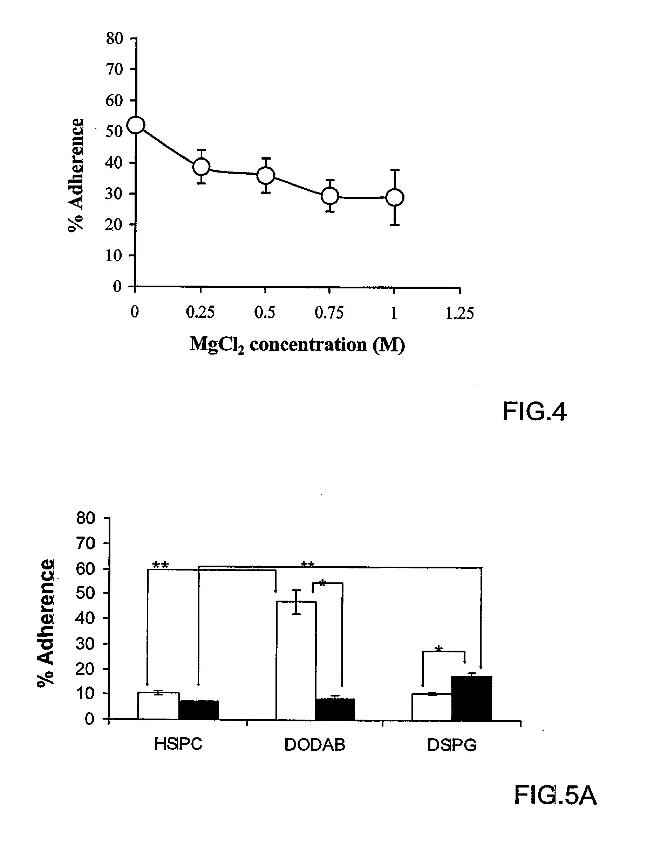

**Fig. 4** is a graph showing the effect of increasing concentrations of MgCl₂ on the % attachment of cationic liposomes (DODAB, 22 mol% of total lipids) to the epithelium of the healthy rat colon after 15 min of incubation in colon sacs. Shown are the averages of four different studies ± SEM.

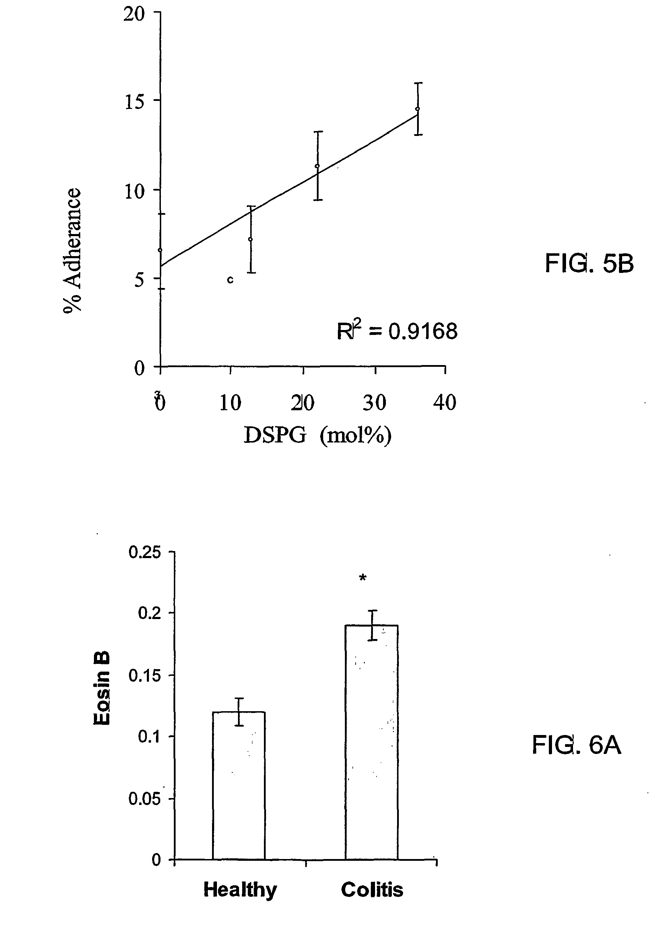

**Figs. 5A-5B** are graphs showing the % adherence of neutral (HSPC), cationic (DODAB, 22 mol% of total lipids) and anionic (DSPG, 22 mol% of total lipids) liposomes (800±50 nm) to healthy (white columns) and inflamed (gray columns) epithelium of the rat colon, as measured 75 min after incubation in colon sacs (**Fig. 5A**) and the effect of charge density (as expressed by the mol% of DSPG of total lipids) on the % adherence of the anionic liposome to the epithelium of the inflamed colon of the rat (**Fig. 5B**). * denotes p<0.001 when compared to healthy group and ** denotes p<0.001 when compared to HSPC group. Shown are the averages of six different studies ± SEM.

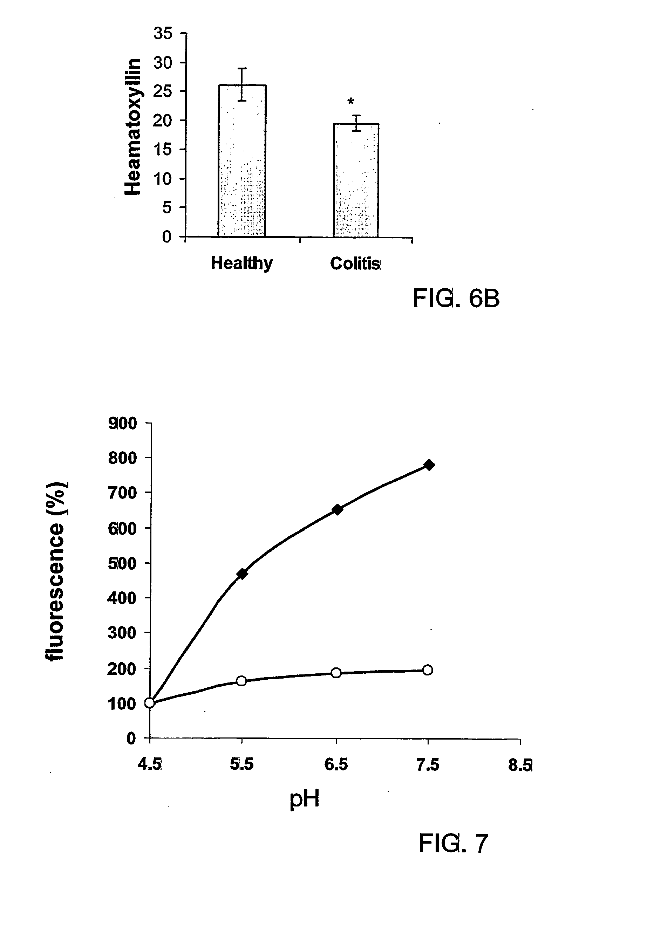

**Figs. 6A-6B** are bar graphs showing the % attachment of Eosin B (mcg/cm²) (**Fig. 6A**) and Hematoxylin (mcg/cm²) (**Fig. 6B**) to healthy and inflamed colonic epithelium of the rat. Shown are the averages of three different studies ± SEM. * denotes *p* < 0.05 when compared to healthy group.
**Fig. 7** is a graph showing the change (increase) in fluorescence (expressed as % of initial fluorescence at pH 4.5) of encapsulated (-◆-) or free (-O-) FITC-SOD with pH.

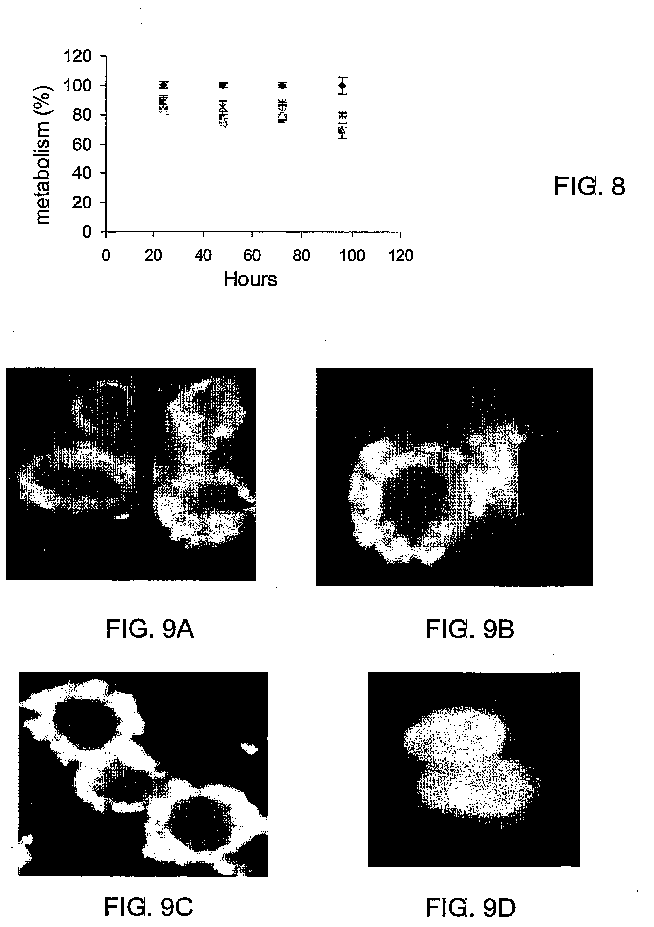

**Fig. 8** is a graph showing the toxicity (relative metabolism) of the cationic liposomes, containing no DODAB (-◆-, control), 19 (-■-), 56 (-▲-), 168 (-x-) and 503 (-∗-) microMolar of DODAB as analyzed by MTT test in HT-29 colorectal adenocarcinoma cells, as a function of time. Shown are the means of triplicate data ± SD.
**Figs. 9A-9D** are confocal fluorescence images of HT-29 cells after incubation with Rhodamine-labeled liposomes containing FITC-SOD for 1 (**Fig. 9A**), 2 (**Fig. 9B**) and 4 (**Fig. 9C**) hours. DAPI stain of HT-29 cells containing FITC-SOD cationic liposomes is also shown (**Fig. 9D**).

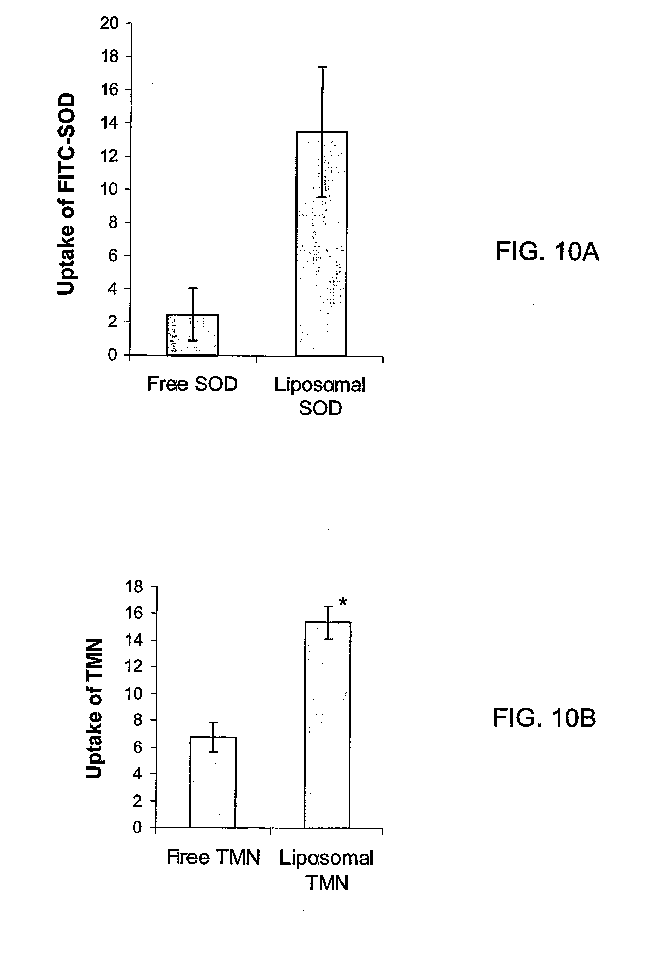

Figs. **10A-10B** are bar graphs showing tissue uptake (% of initial amount/mg tissue protein) of free and liposomal FITC-labeled SOD (FITC-SOD) (**Fig. 10A**) and TMN (**Fig. 10B**) by the epithelium of intestinal sacs from the rat jejunum as expressed by % of initial amount in the bathing solution. Shown are the results of four different experiments ± SEM. * denotes *p* < 0.05 compared with the un-encapsulated group.

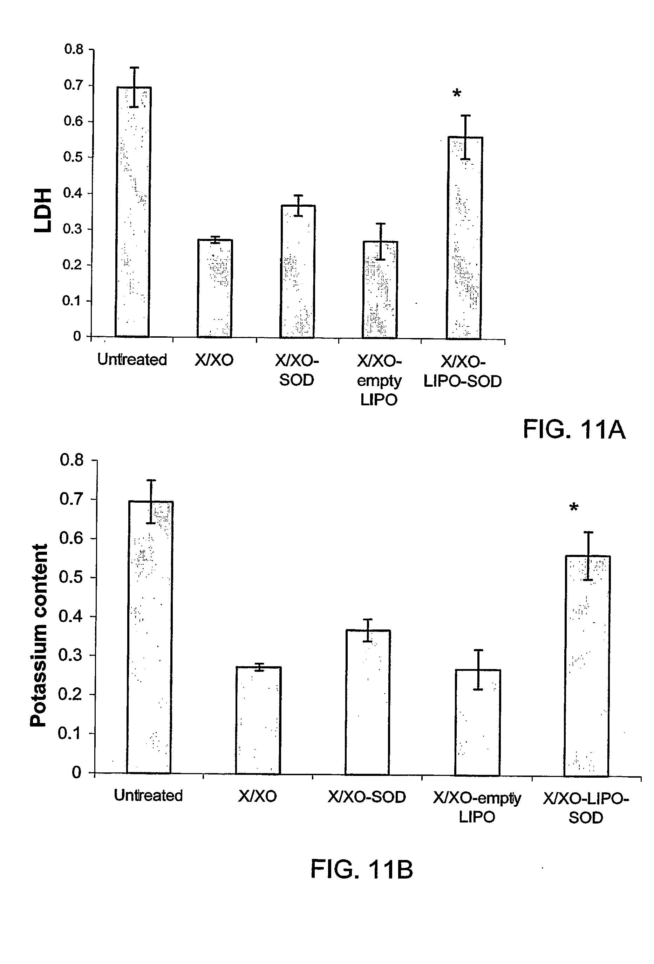

**Figs. 11A-11B** are bar graphs showing tissue LDH activity (Fig. 11A) and tissue content of potassium (Fig. 11B) of treated and untreated healthy jejunal mucosa. Specifically, **Fig. 11A** shows tissue LDH activity (expressed in U/mg tissue) of healthy jejunal mucosa ("**Untreated**") or mucosal tissues treated with xanthine/xanthine oxidase -FeSO₄ mixture followed by saline perfusion ("**X/XO**"), native SOD perfusion ("**X/XO-SOD**"), empty liposomes perfusion ("**X/XO-empty lipo**") and SOD loaded cationic liposomes ("**X/XO-Lipo-SOD**"). **Fig. 11B** shows tissue content of potassium (expressed in µmol/mg tissue) of healthy jejunal mucosa ("**Untreated**") or mucosal tissues treated with xanthine/xanthine oxidase -FeSO₄ mixture followed by saline perfusion ("**X/XO**"), native SOD perfusion ("**X/XO-SOD**"), empty liposomes perfusion ("**X/XO-empty lipo**") and SOD loaded cationic liposomes ("**X/XO-Lipo-SOD**"). Shown are the results of four experiments ± SEM. * denotes *p* < 0.05 compared with the X/XO group.

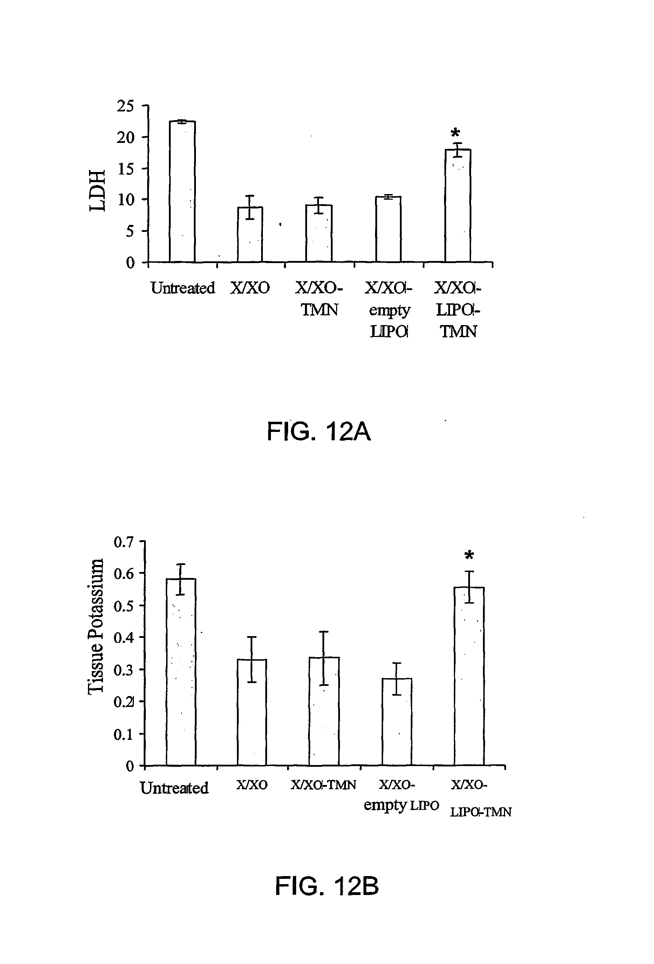

Figs. **12A-12B** are bar graphs showing tissue LDH activity (Fig. 12A) and tissue content of potassium (Fig. 12B) of untreated and treated healthy jejunal mucosa. Specifically, **Fig. 12A** shows tissue LDH activity (expressed in U/mg tissue) of healthy jejunal mucosa ("**Untreated**") or mucosal tissues treated with xanthine/xanthine oxidase -FeSO₄ mixture followed by saline perfusion ("**X/XO**"), TMN perfusion ("**X/XO-TMN**"), empty liposomes perfusion ("**X/XO-empty lipo**") and TMN loaded cationic liposomes ("**X/XO-Lipo-TMN**"). **Fig. 12B** shows tissue content of potassium (expressed in µmol/mg tissue) of healthy jejunal mucosa ("**Untreated**") or mucosal tissues treated with xanthine/xanthine oxidase -FeSO₄ mixture followed by saline perfusion ("**X/XO**"), TMN perfusion ("**X/XO-TMN**"), empty liposomes perfusion ("**X/XO-empty lipo**") and TMN loaded cationic liposomes ("**X/XO-Lipo-TMN**"). Shown are the results of four experiments ± SEM. * denotes *p* < 0.05 compared with the X/XO group.

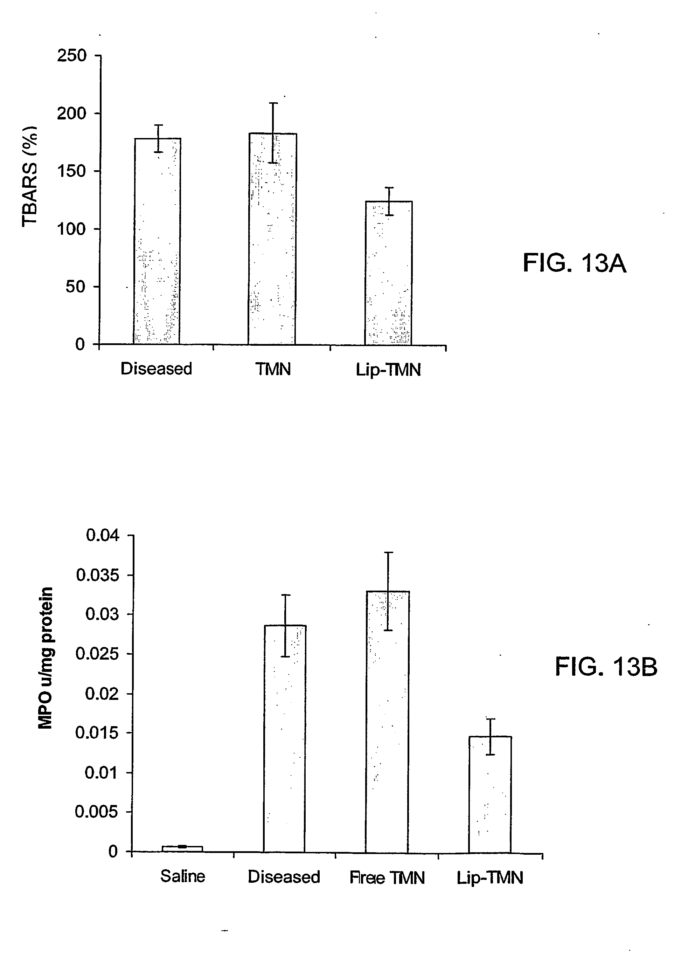

**Figs. 13A-13B** are bar graphs showing the improved anti-inflammatory effect of liposomal TMN (Lip-TMN), compared with the non-liposomal (Free TMN) drug on the colonic epithelium of colitis-induced rats after 3-days treatment, as expressed by tissue TBARS concentration (% of original) (**Fig. 13A**) and MPO (U/mg protein) activity (**Fig. 13B**). Shown are the mean results ± S.E. (n=3), compared with non-treated (Diseased) group and saline treated control.

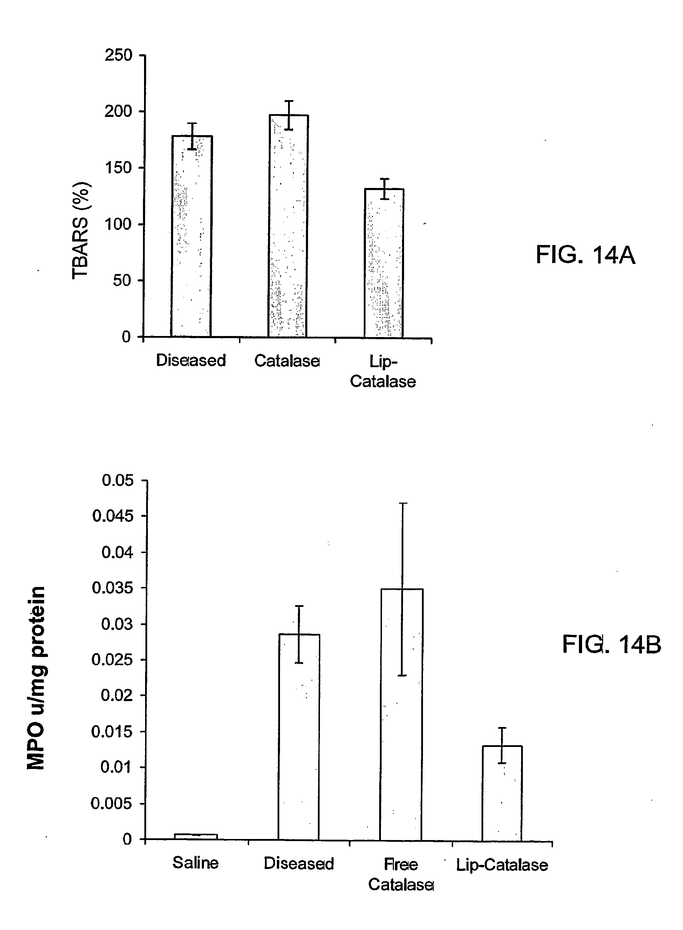

**Fig. 14A-14B** are bare graphs showing the improved anti-inflammatory effect of liposomal catalase (Lip-catalase), compared with the non-liposomal (Catalase) drug on the colonic epithelium of colitis-induced rats after 3-days treatment, as expressed by tissue TBARS concentration (% of original) (**Fig. 14A**) and MPO (U/mg protein) activity (**Fig. 14B**). Shown are the mean results ± S.E. (n=3), compared with non-treated (Diseased) group and saline treated control.

### DETAILED DESCRIPTION OF THE INVENTION

The most important potential of oral particulate drug carriers has been shown to be at the level of the intestinal mucosa since the extent of particulate uptake is still questionable and involves mostly adsorption and some absorption processes [E. Mathiowitz, et al. Nature 1997, 386(6623):410-414]. Thus, much activity has been focused in the area of oral, mucosal immunization, using biodegradable particles [van der Lubben, I. M. et al. Adv. Drug Deliv. Rev. 2001, 52:139-144].

The present invention is based on the use of electrical surface charge as a docking tool to bring lipid assemblies in the vicinity of a diseased, e.g. inflamed epithelium of the mucosa, for executing a desired medical procedure.

Surprisingly, differences in the attachment properties of charged lipid assemblies, such as liposomes, were found, when examined in healthy or inflamed mucosal tissues of rat colon. Specifically, positively-charged liposomes adhered to a healthy mucosa significantly better than anionic or neutral liposomes.

The invention concerns the use of negatively charged liposomes loaded with an active ingredient that is not covalently bound to said liposomes in the manufacture of a medicament for treatment of a disease or disorder associated with inflammation of the mucosa of the gastrointestinal (GI) tract selected from ulcerative colitis, Crohn's disease and colitis.

According to the invention, charged lipid assemblies are used as carriers for targeting the mucosa (epithelium). The targeting is achieved by the differential adhesion of normal (healthy) and diseased (e.g. inflamed) mucosa by, respectively, cationic and anionic lipid assemblies. The successful targeting is preferably used for the local (topical) treatment of disorders confined to the mucosa, in both tissue and enterocyte levels.

The target mucosa according to the invention concerns all that lines body passages and cavities which communicate directly or indirectly with the exterior, including the alimentary, respiratory, and genitourinary tracts. However, according to one embodiment, the target mucosa is that lining the gastrointestinal tract (GIT), including intestinal mucosa, small bowel mucosa, large bowel mucosa or the mucosa in the rectum. More preferably, the invention concerns the intestinal mucosa.

With respect to treatment of GI mucosa, one embodiment concerns diseases, disorders and conditions of the GIT resulting from inflammation. Inflammation can be chronic or acute, or can alternate between the two stages. GIT Inflammation refers to the condition of inflammation occurring or threatening to occur in any portion of the GIT, from mouth to anus. Inflammation of the GIT may be due to etiologies as diverse as infection, reaction to drugs or other foreign (irritating) substances, or to diseases such is Inflammatory Bowel Disease (Crohn's disease; ulcerative colitis). Thus, according to this embodiment, by virtue of their mucosal attachment properties charged lipid assemblies, loaded with active ingredients having an a therapeutic effect, are able to release their drug load in close proximity to the site of injury (e.g. inflammation), thereby increasing drug treatment efficacy. It was shown that the lipid assemblies were also able to enter the enterocytes (epithelium cells) and react immediately with inflammation mediators.

Other diseased states in the GIT include, without being limited thereto, motility disorders, primarily in irritable bowel syndrome (IBS) and malignant processes, such as pre-metastatic colon cancer as well as colon carcinoma, familial adenomatous polyposis. For the treatment of, e.g. IBS the lipid assemblies may be loaded with antispasmodics drugs that control colon muscle spasms and help with diarrhea and pain. Typical examples include 5-HT3 receptor antagonist alosetron hydrochloride and the 5-HT4-receptor agonist (serotonin mimic) tegaserod maleate.

The cause of the different diseases and disorders of the GI may vary. According to one embodiment, the GI state may result from inflammation. To this end, the active ingredient loaded onto the lipid assemblies is one of a variety of agents known in the art to have a therapeutic effect on inflammation processes. Typical, non-limiting examples include steroids, salicylates, COX-2 inhibitors, anti-TNFα drugs and antioxidants.

According to another embodiment, the GI state may result from long term or short term oxidative stress. To this end, the active ingredient loaded onto the lipid assemblies is one of a variety of agents known in the art to have a therapeutic effect on oxidation processes. Typical, non-limiting examples, include antioxidants such as tocopherol, free radicals scavengers, SOD and SOD mimics, catalase or therapeutic reducing agents.

According to yet another embodiment, the GI state may result from motility disorder. According to yet a further embodiment, the GI state may result from malignant processes. To this end, the active ingredient loaded onto the lipid assemblies is one of a variety of cytotoxic or cytostatic agents known in the art to have a therapeutic effect on hyperproliferation conditions within the GI.

The cationic lipids may be monocationic or polycationic (or combination of same), synthetic, semi-synthetic or naturally occurring lipids. Cationic lipids (mono and polycationic) typically have a lipophilic moiety, such as a sterol, an acyl or diacyl chain, and where the lipid has an overall net positive charge. Preferably, the head group of the lipid carries the positive charge. The cationic lipids can be divided into four classes: (i) quaternary ammonium salt lipids (e.g. DOTMA (Lipofectin^{™}) and DOTAP) and phosphonium/arsonium congeners; (ii) lipopolyamines; (iii) cationic lipids bearing both quaternary ammonium and polyamine moieties and (iv) amidinium, guanidinium and heterocyclic salt lipids.

Exemplary of mono cationic lipids include 1,2-dioleyloxy-3-(trimethylamino) propane (DOTAP); cholesterol, dioctadecylmethylammonium bromide (DODAB), N-[1-(2,3,-ditetradecyloxy)propyl]-N,N-dimethyl-N-hydroxyethylammonium bromide (DMRIE); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3β[N-(N',N'-dimethylaminoethane) carbamoly] cholesterol (DC-Chol); and dimethyldioctadecylammonium (DDAB). Additional cationic lipids may be found in http://www.avantilipids.com (transfection reagents therein) incorporated herein by reference.

Non-limiting examples of polycationic lipids include a lipophilic derivatized with a cationic peptide, such as polylysine or other polyamine lipids. other polycationic lipid include, for example N-[2-[[2,5-bis[(3-aminopropyl)amino]-1-oxopentyl]amino]ethyl]- N,N-dimethyl- 2,3-bis[(1-oxo-9-octadecenyl)oxy]-1-propanaminium, (DOSPA), N-(2-hydroxyethyl)-N,N-dimethyl-2,3-bis(tetradecyloxy)-1-propanaminium), and N-palmitoyl D-erythro sphingosyl-1-carbamoyl spermine (CCS).

Non-limiting examples for anionic lipids include 1,2-distearoyl-*sn*-glycero-3-[phospho-rac-(1-glycerol)] (DSPG), hydrogenated soy phosphoglycerol (HSPG) and other as detailed in http://www.avantilipids.com.

The lipid assemblies according to the invention may further comprise a lipid carrier. According to one preferred embodiment the lipid assemblies form vesicles. To this end, the lipids carriers are preferably vesicle forming lipids. One preferred example for vesicles are the liposomes. The vesicle forming lipids are preferably ones having two hydrocarbon chains, typically acyl chains, and a head group, either polar or nonpolar. There are a variety of synthetic as well as semi-synthetic vesicle-forming lipids and naturally-occurring vesicle-forming lipids, including the zwitterionic phospholipids, such as phosphatidylcholines, and sphingomyelins, the negatively charges phospholipids such as phosphatidic acids, phosphatidylinositols, cardiolipins, etc., all of which have preferably two hydrocarbon chains which are typically between about 14-24 carbon atoms in length, and have varying degrees of unsaturation, including fully saturated. Phospholipids whose acyl chains have varying degrees of saturation can be obtained commercially or prepared according to published methods as described, for example in http ://www.avantilipids.com.

The vesicle forming lipids may also include lipids derivatized with a hydrophilic polymer. Such a hydrophilic polymer provides a surface coating of hydrophilic polymer chains on both the inner and outer surfaces of the liposome lipid bilayer membranes. An example for a hydrophilic polymer chain is polyethyleneglycol (PEG), being available in various molecular weights.

Typically, the ratio between the lipid carrier and the charged lipid will be in the range 0.1 mol% -100mol%. According to one embodiment the range is between 3 mol% -50mol%.

The lipid assemblies may include other lipids, e.g. glycolipids, such as and glycosphingolipids such as cerebrosides and gangliosides.

The amount of the active ingredient loaded onto the lipid assemblies (herein also termed the "***effective amount***") includes an amount effective to provide protection from damage to the GI mucosa caused by any of the conditions described herein. The protection includes prevention from damage to develop as well as for curing a condition. An amount being effective to provide the desired protection can be readily determined, in accordance with the invention, by administering to a plurality of tested subjects various amounts of the active ingredient loaded onto charged lipid assemblies and then plotting the physiological response (for example an integrated *"SS index"* combining several of the therapeutically beneficial effects) as a function of the amount of loaded active ingredient. Alternatively, the effective amount may also be determined, at times, through experiments performed in appropriate animal models and then extrapolating to human beings using one of a plurality of conversion methods. As known, the effective amount may depend on a variety of factors such as mode of administration, the age, weight, body surface area, gender, health condition and genetic factors of the subject; other administered drugs; etc.

The medicament according to the invention may also contain a pharmaceutically acceptable carrier. The term *"**pharmaceutically acceptable carrier**"* in the context of the present invention denotes any one of inert, non-toxic materials, which do not react with the lipid assembly or with the active ingredient and which can be added to formulations as diluents, carriers or to give form or consistency to the formulation.

The carrier also includes substances for providing the formulation with stability, sterility and isotonicity (e.g. antimicrobial preservatives, antioxidants, chelating agents and buffers), for preventing the action of microorganisms (e.g. antimicrobial and antifungal agents, such as parabens, chlorobutanol, phenol, sorbic acid and the like), for providing the formulation with an edible flavor or with a color etc.

According to a preferred embodiment, the medicament is in a form suitable for oral or nasal administration. Preferred formulations include liquid, aqueous dispersions, packed in capsules resistant to degradation in the stomach or in proximal parts of the small intestine (e.g. enteric coated capsules lined with various thicknesses, in accord with the desired location of degradation), or enemas or foam-enemas, liquid drops for nasal use and nasal spray. It should be noted that in the context of the present invention, injection of the lipid assemblies and the active ingredient loaded thereon, is preferably excluded.

Finally, the invention concerns the use of charged lipid assemblies for the preparation of a medicament for a medical procedure for treatment or prevention of a disease or disorder of the GI mucosa, wherein for preventing a disease or disorder of the mucosa, positively charged lipid assemblies are used and for treating a disease or disorder of a mucosa negatively charged lipid assemblies are used.

In the following specific examples, three different positively charged lipids were mixed with the zwitterionic lipid hydrogenated soy phosphatidylcholine (HSPC) and the neutral cholesterol to form cationic liposomes: DOTAP, DODAB (both having a single quaternary amine as the cationic group) and DC-cholesterol (having a single tertiary amine as the cationic group). DOTAP and DODAB were found to poses a similar zeta potential at the entire pH range while DC-Cholesterol had a similar Zeta potential values as DOTAP and DODAB at pH below 7 (**Table 1**). Consequently, the mucosal attachment properties of the three types of cationic liposomes to healthy epithelium were approximately similar (**Figure 1**), with DODAB being superior, and increased with increasing level of cationization (**Figure 2**). This finding indicates that the attachment depended primarily on the charge density of the cationic liposomes.

The type of charge, its density, and the liposome size/lamellarity affect their adhesion to either healthy or inflamed epithelium of the rat colon. 100-nm unilamellar vesicles adhered faster and to a larger extent than 800-nm vesicles. However, the difference in adhesion intensity diminished with contact time (slightly better adhesion results for 800-nm vesicles was reached within 75 min, **Figure 3**). A straightforward explanation for these adhesion property differences is the improved distribution, accessibility, and diffusion of the smaller liposomes in the crypt area within a short period of time. Therefore, large liposomes may be favored, unless the site of action is in the colonocyte cytoplasm. **Figure 3B** shows that diminishing the difference in mucoadhesion with time occurred when the cationic liposomes contained relatively high mol% of cationic lipid (36 mol% DODAB). At lower DODAB concentrations (13 mol%) longer incubation times are required to improve the extent of liposome attachment.

The observation that increase in the charge density increased the attachment of the cationized liposomes could be explained by **Figure 4**, which summarizes the effect of increasing concentrations of MgCl₂ on the attachment of cationic liposomes to the colonic epithelium.

The most interesting finding was the one observed with liposomal attachment to injured tissues. When the healthy epithelium was replaced with inflamed epithelium in the colonic sac preparations, anionic liposomes adhered better to the surface of the mucosa (**Figure 5A**). Anionic liposomes adhered better to DNBS-induced epithelium than both cationic and neutral liposomes in a charge (relative amount of DSPG (**Figure 5B**))-dependent manner. A confirmation study was performed with two charged dyes having opposite charges, the anionic dye eosin B and the cationic dye hematoxylin. Its results verified the fmdings of the experiments with the liposomal attachment (**Figure 6**). It should be realized that these differences might be changed if the drug is membrane bound (or part of the membrane) and highly ionized.

The lipid assemblies according to the invention are preferably inert substances, i.e. which do not *a priori* provide a statistically significant therapeutic effect on the healthy or diseased mucosa when provided to the mucosa alone. The combination of the *a priori* inert lipid carrier and the active ingredient provide an elevated therapeutic effect, i.e. an effect which is greater than that provided by the active ingredient alone or in combination with another type of carrier.

The active ingredient varies depending on the selected medical procedure to be executed. As indicated above, the medical procedure may be a preventative medical procedure, i.e. a treatment of a healthy mucosa, for the purpose of preventing a condition from developing. To this end, the active ingredient is loaded onto positively charged lipid assemblies. Alternatively, the medical procedure may be a therapeutic treatment, for the purpose of healing a condition which has already developed. To this end, the subject is treated with negatively charged lipid assemblies loaded with the active ingredient. In both aspects, the active ingredient may be the same. Furthermore, according to the invention, the medical procedure may be comprised of a combination of treatment and prevention, i.e. the subject is administered with negatively charged as well as positively charged lipid assemblies loaded with an active agent, the administration being according to a specific schedule of treatment as determined by considerations available to the physician.

According to one specific embodiment, the medical procedure of the invention is designed for managing gastrointestinal diseases and disorders. Gastrointestinal diseases and disorders include, without being limited thereto, inflammatory, infectious, gastrointestinal motility disorders, gastroesophageal reflux disease (GERD), chronic intestinal pseudo-obstruction (or colonic pseudo-obstruction, disorders and conditions associated with constipation as well as other conditions known to gastroenterologs. More specifically, the gastrointestinal diseases and disorders include, without being limited thereto, inflammatory bowel disease (IBD) including ulcerative colitis, Crohn's disease, peptic ulcer disease including gastric ulceration and duodenal ulceration, ileitis, colitis, ileocolitis, ulcerative proctitis, irritable bowel syndrome, gastroenten'tis, diverticulitis, diverticulosis, reflux, ulcer, gastritis, dyspepsia, nausea, abrasion to gastrointestinal tract, heart bum, hiatal hernia, gastrointestinal abscess, aralytic ileus and diarrhea, constipation associated with use of opiate pain killers, post-surgical constipation, and constipation associated with neuropathic disorders and combinations thereof.

The active ingredient may be selected from agents known and commercially available and will depend on the desired therapeutic procedure. For example, for the treatment of inflammatory conditions, such as inflammatory bowl, steroidal and non-steroidal anti-inflammatory drugs may be loaded onto the lipid assemblies according to the invention. Non-limiting examples include corticosteroids such as Prednisone, Prednisolone, methylprednisolone, methylprednisolone succinate (sodium salt) and Budesonide as well as derivatives of 5-aminosalicylic acid, e.g. Sulfsalazine. Mesalamine (5ASA), Olsalazine and Balsalazide, antibiotics such as Metronidazole Ciprofloxin, Probiotics and other drugs known collectively as "*immunosuppresives*" or "*immunomodulators*" e.g. Cyclosporin A, Azathioprine, Methotrexate and 6-Mercaptopurine.

Alternatively, for the treatment or prevention of conditions resulting from oxidative stress, antioxidants may be used as the active ingredient. Non-limiting examples of anti-oxidants include tempamine (TMN) (which may also be used for treatment of mucosal inflammation), salicylates such as 5-aminosalicylate (5-AS) or 5-aminosalicylic acid (5-ASA) prodrugs (e.g. sulfasalazine) or steroids.

The lipid assemblies loaded with the active ingredient were provided to an animal model for *in vivo* studies. To this end, oxidative insult in the jejunal mucosa of rats was induced by perfusing the small intestine with a mixture of chelated ferrous sulfate, hypoxanthine and xanthine oxidase. This combination produced hydroxyl radicals in a site-specific manner, similar to the way neutrophils cause damage in inflammation processes, or as in pathological conditions (e.g. anoxia caused by ischemic reperfusion), when xanthine dehydrogenase (which, under normal conditions converts hypoxanthine to uric acid) is converted to xanthine oxidase. The metabolism of hypoxanthine by xanthine oxidase results in the release of superoxide anions.

In a first series of assays, the preventative effect of lipid assemblies according to the invention, loaded with anti-oxidants was determined. Localization of an antioxidant enzyme in the preventative assay was accomplished by entrapping it in cationic lipid assemblies. Specifically, liposomes cationization with DODAB were employed, which were *inter alia,* found to be well tolerated by the HT-29 adenocarcinoma cell line, for 80 hours (**Figure 8**). For the preparation of the lipid assembly, cholesterol was added to the lipid combination, which further stabilized the membrane and reduced premature release from the assembly. The use of charged lipids, such as DODAB, further increases the stability of the liposomes by decreasing potential aggregation and eventual fusion of the vesicles. Entrapment of the active ingredient, superoxide dismutase (SOD) inside the liposomes was verified (**Figure 7**) and the possibility that it was adsorbed to the liposomes surface was excluded.

In addition to SOD, the entrapment and preventative effect of loaded Tempamin (TMN) was examined. The use of liposomal TMN and SOD significantly increased the ability of the two antioxidants to protect the rat jejunum against the induced oxidative stress. As control, empty liposomes were perfused in the same experimental protocol and showed no effect (**Figures 11A**-**11B,** **Figures 12A-12B**). In this case, the adhesion of the liposomes to the jejunal mucosa leads to an increase of tissue SOD activity and its enrichment with TMN (**Figures 9**). This increase in residence time is important because even with local administration, the antioxidants are still exposed to rapid removal processes. In addition, the liposomes potentially provide protection for the SOD against proteolysis, for TMN against oxidation or reduction and for both molecules - against premature scavenging by mucin components.

As for TMN, an SOD-mimic, which was shown to neutralize both intracellular and extracellular superoxide, with no preference to the time of administration (prior or after oxidative insult), delivery tools (e.g. liposomes) are highly important, especially because it is a small molecule with the capability of diffusing easily among cellular and tissue components. Anchoring to the site of injury is important due to its mode of action which, unlike other antioxidants that act in a sacrificial mode, provides protection in a (constant) catalytic way. Indeed, **Figure 9** shows that free TMN tissue uptake was 3-fold higher than free SOD uptake, most probably because of its molecular dimensions (mw 171 compared to 33,000) and hence better penetration through the mucus layer, while for TMN amphiphacy and primary amino group enable its superior uptake into the cells.

Internalization studies of liposomal FITC-SOD (**Figures 10A-10C**) clearly show that the cationized liposomes entered the HT-29 cells within the first hour of incubation. Accumulation increased over 4 hours. DAPI staining (**Figure 4D**) showed that the liposomes voyage ended at the cytosol. They could not be found in the cell nucleus within 4 hours.

An interesting question is whether positioning SOD inside the cell is imperative for the antiinflammatory action. The paradigm is that, in inflammatory processes, ROS are generated in interstitial spaces by circulating neutrophils that consume higher amounts of oxygen when activated. Due to their short biological half-life and the non-specific manner of their action, it is expected that they are active at neighboring cellular structures (cell membrane). Previous work of others reported that after exposure to oxidative stress, the survival rate of colonic epithelial cells from the rabbit decreased significantly when intracellular SOD was deactivated by diethyldithiocarbamate. However, internalization is not enough. It should be accompanied by a constant input into the site of injury. It has now been accomplished that by using "sticky" (cationic) liposomes. The ability of the liposomes to stay in the vicinity of the insulted mucosa clearly made them effective therapeutic tools. This is demonstrated by the TMN case. Nitroxide radicals readily cross cell membranes. However, TMN solution was unable to prevent oxidative insult even after intimate contact with the injured jejunal mucosa (**Figures 12A-12B**). It was only after being incorporated into cationized liposomes, that the TMN expressed therapeutic effect because of the much larger residence time at the site of action (**Figure 9**). Yet, it should be recognized that when cationic liposomes adhere to the mucosa of the intestine, they do so, most probably, by sticking to the mucus lining which covers the epithelium first. Indeed, reduction of whole mucins in the gut sacs preparation with dithiothreitol (DTT), prior to incubation with the liposomes, decreased the amount of liposomes adhering to the intestine (data not shown).

Both antioxidants, although acting in different manners, merit from the close proximity to the mucosal tissues, where the entire amount (dose) is directed for the therapeutic action needed with minimal loss in systemic compartments of the body

### SPECIFIC EXAMPLES

### Materials and Methods

Hydrogenated soybean phosphatidylcholine (HSPC) (iodine value 3) was obtained from Lipoid (Ludwigshafen, Germany). 1,2-dioleoyltrimethylammonium-propane (DOTAP), 1,2-dioleolyl-*sn*-glycero-3-phosphoethanolamine-N-(lissamine Rhodamine B) sulfonyl ammonium salt (PEA-Rhod) and 1,2-diolcoyl-*sn*-glycero-3-phosphoethanolamine-*N-*carboxyfluorescein (CF-PE), 3-β-[*N*-(*N*',*N'*- dimethylaminoethane)carbamoyl]-cholesterol (DC-Chol), and 1,2-distearoyl-*sn*-glycero-3-[phospho-*rac*-(1-glycerol)] (DSPG) were obtained from Avanti Polar Lipids (Alabaster, AL, USA). Cholesterol, dioctadecyldimethylammonium bromide (DODAB), dinitrobenzenesulfonic acid (DNBS), hematoxylin, eosin B xanthine oxidase, hypoxanthine, 4-amino tempol (tempamine, denoted as TMN), Triton X-100, fluoresceine isothiocyanate (FITC), 3-(4,5-dimethylthiazol-2-yl)-2,5-diphenyltetrazolinium bromide) (MTT); 4',6-diamidine-2'-phenylindole diHCl (DAPI) and superoxide dismutase (SOD) were obtained from Sigma (Sigma Chemical Co., St. Louis, MO, USA).

All other chemicals were of analytical grade unless otherwise stated in the text.

### Liposome Preparation

*Neutral fluorescent liposomes:* Appropriate amounts of lipids (containing 16 mM HSPC and 12 mM cholesterol) and 0.5mol% headgroup labeled 1,2-dioleoyl-*sn*-glycero-3-phosphoethanolamine-*N-*carboxyfluorescein (CF-PE) were weighed, dissolved in tert-butanol, and lyophilized overnight. The lyophilized bed of lipids was hydrated using distilled water at 60°C, a temperature above the HSPC gel-to-liquid crystalline phase transition temperature (52°C), and sonicated by a probe sonicator (Microson™, UL Laboratory Equipment, USA) till a translucent dispersion was achieved. The obtained liposomes were then freeze-thawed three times, and lyophilized overnight. The lyophilized bed was then hydrated by L-histidine buffer (5 mM in normal saline) pH 6.5, to the appropriate volume. In this formulation, HSPC served as the main liposome-forming lipid and was selected due to its high solid-ordered to liquid-disordered (SO↔LD) phase transition temperature (52°C). Cholesterol was included in the liposomal lipid so as to obtain a lipid membrane in a liquid-ordered (LO) form. This led to the improved physical stability of the liposomes during storage, and in the body [Barenholz Y Prog. Lipid Res. 2002 41, 1-5]. Liposomes composed of HSPC and cholesterol have a record of good performance in various applications [Lasic D.D. Trends Biotechnol. 1998 16, 307-321] because of their high chemical and physical stability, contributing to good drug retention with minimal leakage [Haran, G. et al. 1993 Biochim. Biophys. Acta 1151, 201-215].

*Cationic liposomes:* were prepared as described for the neutral liposomes, except for the addition of the cationic lipid DODAB at the desired level (13, 22, or 36 mol% of total lipids). In separate studies, cationic liposomes containing 22 mol% of DOTAP or 22 mol% of DC-cholesterol were also prepared.

*Anionic liposomes:* were prepared as described for the neutral liposomes, except for the addition of the anionic lipid DSPG or HSPG at the desired level (13, 22, or 36 mol% of total lipids).

Throughout the study, all liposomes were extruded above their Tm (at 65°C), 11 times through 800-nm-pore-size polycarbonate filters using the LiposoFast syringe extruder (Avestin, Ottawa, ON, Canada) to prepare sized multilamellar liposomes (MLVs). In some cases this was followed by extrusion through a 100-nm-pore-size polycarbonate filter, to prepare 100 nm unilamellar vesicles. The dimensions of the liposomes were analyzed by submicron particle sizer (Coulter, Luton, UK). For each batch, a size-distribution curve was plotted (not shown). Thus, the average size of the large liposomes was 800±50 nm and that of the small liposomes was 100±27 nm (mean value ± S.D.). The liposomes maintained these measured dimensions throughout the course of the study.

*Zeta potential:* of the liposomes was measured, after dilution (1/100) in a 0.01 M unbuffered NaCl solution, by a Zetasizer 3000 HS system (Malvern, England).

*Liposomal charge:* was assessed by zeta potential analysis after dilution (1/100) in a 0.01 MNaCl (Zetasizer 3000 HS, Malvern, England).

### Animals, maintenance anesthesia and euthanasia

Male Sprague-Dawley or Male Sabra rats (220-250 g) were obtained from the Animal Farm of Hadassah Medical Center at The Hebrew University of Jerusalem. They were kept under constant environmental conditions (22°C, 12-h light/dark cycles) and fed with standard laboratory chow and tap water. All animal studies were conducted in accord with the Principles of Laboratory Animal Care (NIH publication #85-23, revised 1985). The Mutual Committee of Hadassah University Hospital and the Faculty of Medicine for Animal Welfare approved the study protocol. Anesthesia was performed by an intraperitoneal injection of 100 mg/kg body weight of Ketamine (Ketaset, Fort Dodge, USA). Euthanasia of the anesthetized rats was carried out by chest wall puncturing.

### Induction of Experimental Colitis

Twenty-four hours prior to colitis induction the rats were deprived of food, but allowed free access to water. The water contained 10 mg/l of the laxatives sennoside-A and sennoside-B (X-Prep Liquid®, Rafa Pharmaceuticals, Israel) and sucrose (200 g/l) With the rats under light ether inhalation anesthesia, colitis was induced by intracolonic administration of 30 mg of dinitrobenzensulfonic acid (DNBS) dissolved in 1 ml of an ethanolic solution 25% (v/v). The solution was instilled slowly over 20 s via a flexible, perforated foley catheter, which was then immediately removed, leaving the rats in an upside down position for another 40s.

In separate studies, rats were dosed intra-colonically with 1 ml of free or liposome encapsulated catalase (activity: 9,600U), or 1 ml of free or liposome encapsulated TMN (5 mM). The administration was carried out 1 hour following colitis induction and repeated every 12 hr over three days. The rats were sacrificed 4 days after colitis induction.

### Inflammation Severity Characterization

Inflammation was quantified macroscopically and by monitoring myeloperoxidase (MPO) activity, as described elsewhere [Krawisz J.E. et al. Gastroenterology 1984, 87:1344-1350; Grisham M.B., Methods Enzymol 1990, 186:729-742]. Briefly, a specimen from the colonic mucosa was taken and homogenized on ice. 0.5 ml of the homogenized tissue was centrifuged at 25,000g for 5 min. The pellet was dispersed in 0.5 ml of ice-cold 50 mM phosphate buffer, pH 6, containing 0.5 ml of hexadecyltrimethyl ammonium bromide (HTAB). The suspension was frozen-thawed twice, sonicated for 15 s, and centrifuged at 5000g for 5 min. Then 0.1 ml of supernatant was added to 2.9 ml of phosphate buffer, pH 6, containing 0.167 mg/ml o-dianisidine hydrochloride and 5x 10⁻⁴ % v/v of hydrogen peroxide. The rate of change in absorbance was determined at 460 nm over 30 s. Results were expressed as units of myloperoxidase (MPO) activity per gram tissue weight.

Macroscopic evaluation was performed by scoring the damage observed on a 0-5 scale (**Table 1**), as previously described [Blau S. et al. Pharm. Res. 2000, 17:1077-1084]. In the experimental setup described herein the whole length of the colon was inflamed, with intermittent ulcers. Inflamed tissues with a damage score of 3 - 5 were selected for the attachment studies in which the liposomes' adherence was compared in healthy and inflamed tissues. The average value of MPO activity in healthy tissues was 0.02±0.005U/g tissue, while that of the inflamed tissues averaged 5 times higher (at 0.097±0.01U/g tissue).

### Colon Sac Preparation and Adherence Studies

The abdomen of either healthy (control) or colitis-induced rats was cut open and a 10 cm length of from the distal colon was excised and rinsed with PBS, after which 5-cm long sacs were prepared by tying one end with 3/0 silk suture, filling with 0.4 ml of liposomal suspension (diluted x10 in PBS), and tying the other end. The rest of the colon was excised and kept for macroscopic scoring and MPO analysis. The sacs were incubated in 15 ml of PBS, containing 10 mM glucose, at 37°C, in a glass vial on a shaking bath for either 15 or 75 min. At the end of the incubation, the sacs were cut open, rinsed three times by immersion in PBS and weighed. The tissues were homogenized with a Polytron (Kinematica GmbH, Germany) in a solution containing isopropanol: borate buffer pH 9.0 (9:1). One milliliter of the homogenate was centrifuged at 14,000 rpm for 15 min. The level of fluorescence in the supernatant was then measured by a spectrofluorimeter (Perkin-Elmer LS-SB, England); λ excitation = 495, λ emission = 525

### Tissue Staining by Charged Dyes

Colonic sacs, of either healthy or inflamed rat colonic tissues, were prepared as described above and filled, in separate studies, with 0.5 ml of aqueous solutions (containing 2% v/v DMSO) of either of the cationic dye, hematoxylin (1 mg/ml), or the anionic dye, eosin B (0.5 mg/ml). The sacs were incubated in 15 ml of PBS containing glucose (10 mM), at 37°C (glass vial) for 15 min in a shaking bath, after which the sacs were cut open, measured for their surface area (ruler) and rinsed (PBS) three times. The tissues incubated with eosin B were then homogenized in 3 ml of absolute methanol, while the tissues incubated with hematoxylin were homogenized in 3 ml of absolute ethanol. The concentration of dyes in the tissue homogenates was measured spectrophotometrically (Uvikon 933, Kontron Switzerland), at 292 nm (hematoxylin) or 523nm (eosin B).

### TMN encapsulation

TMN was loaded into the liposomes by the ammonium sulfate gradient [Haran et al. **1993** ibid.]. Briefly, lipids where lyophilized from tertiary butanol as above and water (70°C) was added to the lipid film. The resulting liposomes were sonicated to form SUV's. Ammonium sulfate solution (0.25M) was added, and the dispersion was freeze-thawed ten times, after which it was lyophilized overnight. L-histidine buffer was then added to a final concentration of 10mM and 70°C, and the liposomes were extruded at 65°C, 11 times through 400-nm pore-size polycarbonate filters using the LiposoFast syringe extruder (Avestin, Ottawa, ON, Canada) to prepare sized multilamellar liposomes (MLVs). The size distribution of the liposomes was analyzed by a Submicrometer Particle Sizer (Coulter, Luton, UK). For each batch, a size-distribution curve was plotted and the average size of the liposomes was 400 ± 50 nm (mean value ± SD). Free ammonium sulfate was removed by dialysis against normal saline. Existence of the ammonium sulfate gradient was verified by the acridine orange test [Haran et al. **1993**. ibid.]. TMN (10 mM) was added at 70°C and the system incubated for 15 min. Non-encapsulated TMN was removed by dialysis against normal saline, the final extra-liposome pH was brought to pH 6.5 by the addition of L-histidine buffer to a final concentration of 10mM. The encapsulated TMN concentration was measured by electron paramagnetic resonance analysis (EPR) using an 8 point calibration curve of TMN [Krishna M.C. et al. J. Biol. Chem. 1996, 271(42):26026-26031]. The encapsulation yield averaged at 80%.

In the specific example below, TMN was encapsulated into liposomes prepared from HSPC:HSPG:cholesterol in a mole ratio of 16:8:12.

### Catalase encapsulation

Catalase solution (in PBS, between 500 and 5000 units) was added to the lipid cake (lyophilizate) as above, followed by freeze-thawed three times. Liposomes were down-sized to 400nm as above and the free catalase was removed from the liposomes by ultracentrifugation.

### Tagging SOD with FITC

SOD was tagged with FITC to enable the detection of its uptake into the cells. FITC was dissolved in DMSO and SOD was dissolved in sodium bicarbonate buffer pH 9, 0.1M. The two solutions were mixed (magnetic stirrer, I h, room temperature) to form a mole ratio of 1:3 SOD:FITC. Unreacted FITC was separated from the FITC-SOD by dialysis against saline. The FITC-SOD was characterized for its fluorescence spectrum and its number of FITC moiety per molecule of protein. This FITC-SOD was then immediately loaded into the liposomes.

### FITC-SOD encapsulation

The lyophilized bed of lipids (see above) was hydrated in acetate buffer, pH=5.5 and the tagged FITC-SOD solution (0.75 mg/ml) added. The mixture was freeze-thawed three times (60°C). The MLVs obtained were extruded as mentioned above to an average size distribution of 400 nm. Excess of unloaded SOD was removed by ultra-centrifugation and decantation (3 times). FITC-SOD-content in the liposomes was determined by (***a***) activity measurements by the cytochrome-C method [McCord J.M. and Fridovich S.E. J. Biol. Chem. 1969, 22:6049-6055], (***b***) fluorescence measurements and (***c***) protein contents [Bradford M.M. Anal. Biochem. 1976, 72:248-254]. Supernatant FITC-SOD activity measurement verified the results of the liposomal FITC-SOD activity measurements.

### SOD locality within the liposomes

To verify whether the liposomal FITC-SOD was inside the liposomes or adsorbed onto the surface of the lipid layers outside the liposome, FITC-SOD-loaded liposomes or free FITC-SOD were diluted in buffer solutions of the following pH values: 4.5 (citrate buffer, 0.1M), 5.5, 6.5, 7.5 (bicarbonate buffer, 0.1M) and 8.5 (borate buffer, 0.1M). The change in fluorescence intensity was recorded spectrofluorimetrically (Perkin-Elmer LS-SB, Bucks, England). λ emission=525 nm, λ, excitation=495 nm. It was expected that the increase in pH would lead to a concomitant increase in the fluorescence, only when FITC-SOD was subjected to direct contact with the altered pH, namely non-encapsulated, or it was adsorbed on the surface of the liposomes.

### Cytotoxicity studies

The cytotoxicity of the cationic liposomes was assessed in the human colon adenocarcinoma cell line HT-29 using a modified 3-(4,5-dimethylthiazol-2-yl)-2,5-diphenyltetrazolinium bromide) (MTT) assay [Hansen, M. B. et al .J. Immunol. Met., 119:203-210, 1989]. Cells were maintained in DMEM containing 10% FCS and grown at 37°C in 5% CO₂ (v/v) in air. Cells were seeded in 96-well microtitre plates at a density of 10,000 cells per well. Twenty-four hours after plating, increasing concentrations of the cationic liposomes in fresh media were added. The cells were then incubated for additional 24, 48 or 72h. Cellular metabolic activity was assayed by incubating with MTT (0.5mg/ml per well) for the final four hours of the designated exposure period, solubilizing the formed formazan crystals with 10% SDS in 0.01N HCl and monitoring the sample absorbance at 570nm, with a background reference wavelength of 630 nm.

### Internalization of SOD-loaded liposome into HT29 cells

The ability of HT29 cells to internalize SOD-loaded liposomes in culture was examined. HT29 cells (2x10⁴/ 200µl) were seeded onto cover slips contained in 24 well trays. After 24h the cells were exposed to SOD-loaded liposomes for 1, 2 and 4h at 37°C. To determine SOD internalization, FITC-SOD was introduced into non-labeled liposomes. To verify cellular internalization of both SOD and liposomes, FITC-SOD was loaded in headgroup labeled Rhodamine phosphatidylethanol amine liposomes. At the end of incubation the cells were rinsed three times with cold PBS and fixed in 3% paraformaldehyde. Nuclear morphology visualization was enabled by soaking the fixed cells in 4',6-diamidine-2'-phenylindole dihydrochloride (DAPI), 50µg/ml in PBS, for 20-30 min, at RT. After PBS rinsing (three times) the cells were mounted in Mowiol-DABCO mounting media. Cell internalization and cytosol locality (in relation to the nucleus) was examined by confocal microscopy (Zeiss, Gottingen, Germany).

### Colon sac preparation and uptake studies of SOD and TMN

The abdomens of anesthetized healthy Sabra rats were cut open and 10 cm long segments of the distal colon were excised, separated and rinsed with PBS. Colon sacs (5 cm each) were prepared by tying one end with 3/0 silk suture, filling with 0.4 ml of free or liposomal suspension of either TMN or FITC-SOD (diluted x10 in PBS), and tying at the other end.

The sacs were incubated in 15 ml of PBS, containing 10 mM glucose, at 37°C, in a glass vial in a shaking bath for 60 min. At the end of the incubation, the sacs were cut open, rinsed three times by PBS immersion and weighed. For detection of FITC-SOD attachment and uptake (subsequently referred to generally as uptake, as internalization and attachment cannot be discriminated using these methods), the colon sac preparations were homogenized (Polytron, Kinematicas, Berlin, Germany) in a solution containing isopropanol: pH 9.0 borate buffer 9:1. One milliliter of the homogenate was centrifuged at 14,000 rpm for 15 min. The level of fluorescence in the supernatant was then measured by a spectrofluorimeter (Perkin-Elmer LS-SB, Bucks, England) at λ excitation=495, λ emission = 525. For the detection of TMN uptake, tissues were homogenized in water containing 1% v/v TritonX-100. One ml of the homogenate was centrifuged (5000 rpm, 15 min), and TMN concentration was measured by EPR.

### Local induction of oxidative stress in rat jejunum

Hydroxyl radicals were induced in the jejunum (15 cm long segment) of each of four group of anaesthetized Sabra rats, by the perfusion of a mixture of 6mM hypoxanthine, 3U/ml of xanthine oxidase, 10 mM FeSO₄ and 1 mM EDTA in saline over 30 min, using a closed rat jejunal loop system at a rate of 1ml/min, as described elsewhere [Kohen R. et al. J. Biol. Chem. 1992, 267:21349-21354].

In separate studies two groups of rats (4 rats in each group) were perfused with either 35U/ml SOD or liposomal SOD over 20 min. A 10 min saline rinse was then performed, followed by the hydroxyl radicals induction as described above. Similarly, the other two groups (4 rats each) were perfused with 5mM free TMN or liposomal TMN over 20 min. A 10 min saline rinse was then performed, followed by hydroxyl radicals induction as described above. Control studies included (***a***) a group of rats, perfused with normal saline over 30 minutes prior to the oxidative damage induction, (***b***) a group of rats, perfused with normal saline for 20 min (naive control). At the end of each study, the perfused jejunal segment was separated from the anaesthetized animal, the mucosal epithelial layer delicately scraped and homogenized in 5 ml of distilled water on ice.

### Injury quantification

Assuming that tissue insult would cause leakage of enterocyte contents, the induced damage was characterized by measuring tissue (***a***) activity of lactate dehydrogenase (LDH) at 340 nm with pyruvic acid as the substrate and NADH as the electron donor. For this purpose, 1 ml of the tissue homogenate was centrifuged at 5000g for 15 min. LDH activity was measured in the supernatant; (***b***) potassium levels by atomic absorption. All measurements were repeated four times, and the results were normalized to tissue dry weight.

### Tissue Collection

The rats were anesthetized by an IP injection of 100 mg/kg body weight of ketamine (Ketaset, Fort Dodge, USA)] and their colons were exposed through a longitudinal abdominal incision. The distal 10-cm of the colon was removed, cut open, and rinsed with ice-cold PBS, pH 7.4. Ulcerated regions were located, and full thickness of ulcerated tissues was separated with a scalpel from the surrounding inflamed tissues. The tissue specimens were immediately frozen in liquid nitrogen. At a later stage the specimens were homogenized (Polytron, Kinematica GmbH, Germany) in 10 volumes of 0.02M phosphate-buffer, pH 7.4, and stored again at -74°C for further biochemical analysis and inflammatory markers' evaluation.

### Inflammation quantification

### Tissue myeloperoxidase (MPO) activity

The activity of the enzyme MPO is a reliable index of inflammation caused by infiltration of activated neutrophils into the colonic epithelium. Its activity was evaluated according to Grisham *et al.* (2). Briefly, 0.5 ml taken from the frozen, homogenized colonic mucosa was centrifuged at 25,000 g for 5 min. The pellet was dispersed in 0.5 ml of ice-cold 50 mM phosphate buffer, pH 6, containing 0.5 ml hexadecyltrimethyl ammonium bromide (HTAB). The suspension was freeze-thawed twice, sonicated for 15 seconds and centrifuged at 5000 g for 5 min. 0.1 ml of supernatant was added to 2.9 ml of phosphate buffer, pH 6, containing 0.167 mg/ml o-dianisidine hydrochloride and 0.0005 %v/v of hydrogen peroxide. The rate of change in absorbance was determined at 460 nm over 30 seconds. Results were expressed as units of MPO activity per mg of protein (3).

### Measurement of Thiobarbituric acid reactive species (TBARS)

1 ml of the tissue homogenate was centrifuged (5000 rpm, 15 min) and the supernatant was mixed with 2 ml of a mixture of 15% trichloroacetic acid, 0.37% thiobarbituric acid and 0.25 N HCl. The mixture was heated (100°C) for 15 min and then centrifuged. The absorbance of the supernatant was measured at 535 nm.

### Statistical Analysis

Data were analyzed by the Kruskal-Wallis test. The results were expressed as mean values ± SEM. The Mann-Whitney test was then used to identify differences between the groups. A difference was considered to be statistically significant when the *p* value was <0.05. When a difference between the groups was obtained, a Mann-Witney test was used to analyze the significance of the difference between the individual group means (p<0.05).

### RESULTS

The effect of liposome composition on liposome Zeta potential is shown in Table 1.

**Table 1: Liposome composition and Zeta potential (measured in unbuffered NaCl)**

| **Liposome type** | **Lipid composition (mol%)** | **Zeta potential (mV)** |
|---|---|---|
| Neutral | HSPC:Chol | -12 |
| | 57:43 | |
| Cationic | HSPC:Chol:DOTAP | 70 |
| | 45:33:22 | |
| Cationic | HSPC:Chol:DC-Chol | 68 |
| | 45 :3 3 :22 | |
| Cationic | HSPC:Chol:DODAB | 49 |
| | 50:37:13 | |
| Cationic | HSPC:Chol:DODAB | 64 |
| | 45:33:22 | |
| Cationic | HSPC:Chol:DODAB | 76 |
| | 36:28:36 | |
| Anionic | HSPC:Chol:DSPG | -28 |
| | 50:37:13 | |
| Anionic | HSPC:Chol:DSPG | -66 |
| | 45:33:22 | |

The adherence of cationic, anionic, and neutral liposomes (22 mol% of cationic or anionic lipids in the formulations; liposome sizes were 800-1000 nm) to the epithelium of the healthy colon of the rat is shown in **Figure 1**. The amount of liposomes attached to the colonic epithelium was calculated as the relative fluorescence detected in the tissue homogenates. In all cases (three different types of cationic lipids) the adherence of the cationic liposomes was better than the anionic or neutral ones (47.0± 5.0, 36.96±3.98, 33.76±4.21% fluorescence of initial amount per g tissue wet weight ± SEM for liposomes containing DODAB, DOTAP, DC-Chol, respectively, compared with 10.53±0.78 and 11.99±1.98% fluorescence of initial amount per g tissue wet weight ± SEM for liposomes containing DSPG and neutral liposomes, respectively).

The adherence of the cationic liposomes was charge density dependent (expressed as mol% of charged lipids) (**Figure 2**). That is, the higher the amount of cationic lipid in the lipid mixture, the higher the adherence measured as observed in the case of DODAB (11±1.41, 39.08±1.78, 47.0±5.0, 70.0±2.47% fluorescence of initial amount per g tissue wet weight ± SEM. for 0,13, 22, and 36% DODAB- containing liposomes, respectively).

The effect of liposome size on liposome adhesion to the colonic mucosa was also evaluated. A significantly larger fraction of the 100-nm liposomes adhered to the colonic mucosa than the 800-nm MLVs at 15 min incubation time, as shown in **Figure 3A**. However, after 75 minutes, the adhesion of the 800-nm liposomes was better (33.6±1.7 and 36.9±3.7% fluorescence of initial amount per gram tissue wet weight ± SEM for 100-nm liposomes at 15 and 75 min incubation respectively; 20±4.6 and 47±5.0% fluorescence of initial amount per gram tissue wet weight ± SEM for 800-nm liposomes at 15 and 75 min incubation, respectively).

The relationship between the charge density, expressed as mol% of cationic lipid in the liposomal formulation, and the incubation time in the colonic sacs was also tested. **Figure 3B** demonstrates that charge density has a greater effect on liposomal attachment to the tissue than does incubation duration. **Figures 3A** and **3B** show that the effect of incubation time on adhesion was more pronounced in the case of larger liposomes and in those liposomes that contained lesser amounts of DODAB (low cationic charge density).

The nature of the electrostatic interaction between the colonic mucosa and the cationic liposomes (DODAB 22 mol%) was further examined by co-incubation (competition) with elevated concentrations of magnesium chloride. The results, which are summarized in **Figure 4**, show that an increase in the concentration of MgCl₂ (in the range of 0.25-1.0 M) had only a limited effect on the cationic liposome adherence to the colonic mucosa (ratio between DODAB and MgCl₂ concentrations ranging from 1/3000 to 1/12500), indicating that the polyvalency of the cationic liposomes is by far advantageous on bivalent ions, similar to what has been observed with respect to the effect of salts on the interaction between nucleic acids and cationic liposomes, [Hirch-Lerner et al. Biochm.Biophys. ACTA, submitted].

When the attachment of the various types of liposomes was measured in inflamed and healthy tissues, it was found that anionic liposomes adhered better to the inflamed colon than did cationic liposomes (17.8±0.95, 8.5±1.35, 7.05±0.25% fluorescence of initial amount per g tissue wet weight ± SEM for liposomes containing DSPG, DODAB and HSPC, respectively) (**Figure 5A**). Moreover, this attachment to the colitis-induced epithelium was charge-density dependent. Better attachments were observed (6.5±2.11, 7.5±1.89, 11.3±1.91, and 14.5±1.46% fluorescence of initial amount per g tissue wet weight ± SEM for 0, 13, 22, and 36% DSPG-containing liposomes, respectively) (insert of **Figure 5B**) with higher amounts of DSPG in the liposomal lipid mixture. The attachment of the neutral liposomes was found to be similar in both healthy and colitis-induced epithelium. The attraction between the inflamed epithelium and negatively charged groups was verified by the studies involving charged dyes. **Figure 6A** shows that the anionic dye eosin B adhered significantly better to the inflamed colonic epithelium than to the healthy tissue (0.12±0.011 and 0.19±0.012 µg/cm² for healthy and inflamed tissues, respectively). The cationic dye hematoxylin, on the other hand, adhered significantly better to healthy tissues than to inflamed ones (**Figure 6B**) (26.2±2.8 and 19.6±1.36 µg/cm² for healthy and inflamed tissues, respectively).

To identify internalization of the FITC-SOD into the liposomal preparations the loaded liposomes were incubated in buffer solutions of increasing pH values. **Figure 7** shows that while the fluorescence of free FITC-SOD intensified with pH-increase, the fluorescence of the liposomal FITC-SOD almost did not change with pH, indicating that the tagged liposomal enzyme was entrapped in the liposome construct (where the pH was 5.5, resulting a typical fluorescence at that pH) and was not adsorbed to the liposomes surface.

The cationic liposomes were well tolerated by the colorectal adenocarcinoma cell line HT-29, for up to 96 hours, as measured by the MTT assay, summarized in **Figure 8**. The same cell-line was used to characterize cellular internalization of the liposomes. The liposomes were labeled with Rhodamine and loaded with FITC-SOD. Typical examples of visualization, which was performed at 1, 2, and 4 hours are presented in **Figures 9A-9C**. It was observed that FITC-SOD (white) was localized at discrete points within the cell cytoplasm. Rhodamine-labeled phospholipids (gray) were also identified within the cytoplasm, indicating that the HT-29 cells engulfed the entire liposomes. DAPI staining showed that the location of the liposomal SOD was confined to the cytosol, excluding the nucleus (white).

Tissue uptake studies were consistent with the cell line observations. As shown in **Figure 10A** and **10B****,** the liposomal preparations of both SOD and TMN, respectively, were efficiently taken by the epithelium of the rat intestine compared with the native enzyme and free TMN (13.5±3.9 and 15.4±1.2 compared with 2.5±1.6 and 6.8±1.1% uptake of initial amount ± SEM, respectively).

The oxidative damage induced by hydroxyl radicals caused a 3-fold decrease in the activity of tissue LDH and almost 2-fold reduction in the tissue potassium levels in epithelium of the rat jejunum. Pretreatment at the location of injury with native SOD or empty liposomes was unable to prevent this damage. However, perfusing the jejunum with liposomal SOD significantly protected the intestinal tissues against the noxious effects of the hydroxyl radicals. LDH activity was 22.4± 0.2, 8.7:1: 1.8, 11.8±0.3, 10.4±0.3 and 17.8±1.9 U/mg tissue ± SEM for the untreated group, the oxidative damage treated group, the native SOD pretreated group, the empty liposomes pretreated group and the liposomal SOD pretreated group respectively (**Figure 11A**). Potassium levels were: 0.7±0.06, 0.33±0.07, 0.37±0.03, 0.56±0.06 and 0.27±0.05 µmol potassium/mg tissue for the untreated group, the oxidative damage treated group, the native SOD pretreated group, the liposomal SOD and the empty liposomes pretreated group respectively (**Figure 11B**).

Similarly, **Figure 12A** summarizes the advantages of liposomal TMN compared with free TMN in the local treatment of oxidative injury caused by hydroxyl radicals. While free TMN was not able to protect the epithelium against oxidative damage was low, liposomal TMN doubled the protection effect as expressed by tissue activity of LDH and potassium levels. Mucosal LDH activity was 22.4± 0.2, 8.7± 1.8, 9.0±1.3, 10.4±0.3, 17.9±1.1 U/mg tissue ± SEM for the untreated group, the oxidative damage induced group, the free TMN pretreated group, the empty liposomes pretreated group and the liposomal TMN pretreated group respectively. The corresponding mucosal potassium levels were found to be 0.6±0.04, 0.35±0.07, 0.33±0.08, 0.55±0.05 and 0.27±0.05 µmol/mg tissue for the untreated group, the oxidative damage induced group, the free TMN pretreated group, the liposomal TMN and the empty liposomes pretreated group respectively (**Figure 12B**).

In a further assay, TMN was loaded onto negatively charged liposomes (composed of HSPC, HPG and cholesterol as described in the materials and methods) and the anti-inflammatory effect of the loaded liposomes was examined. In particular, after three days of treatment of colitis induced rates the effect of the negatively charged loaded liposomes on the colonic epithelium was examined. **Figures 13A-13B** show the effect of the loaded liposomes as expressed by tissue TBARS concentration (**Figure 13A**) and MPO activity (**Figure 13B**). The therapeutic activity of the negatively charged liposomes loaded with TMN is significant when compared to the free drug or the results with saline treated rats.

The improved anti-inflammatory effect of liposomal catalase, compared with the free (non-liposomal) drug on the colonic epithelium of colitis-induced rats after 3-days treatment was also examined. **Figures 14A-14B** show this effect, as expressed by tissue TBARS concentration (**Figure 14A**) and MPO activity (**Figure 14B**). Also in this case, the superiority of the liposomal drug is evident.

## Claims

1. Use of negatively charged liposomes loaded with an active ingredient that is not covalently bound to said liposomes in the manufacture of a medicament for treatment of a disease or disorder associated with inflammation of the mucosa of the gastrointestinal (GI) tract selected from ulcerative colitis, Crohn's disease and colitis.

2. Use as claimed in Claim 1, wherein the loading of the liposomes with the active ingredient comprises encapsulation, entrapment or electrostatic adsorption of the active ingredient to the liposome, or any combination thereof.

3. Use as claimed in Claim 1 or 2, wherein the medicament is for local treatment of the disease or disorder specified in Claim 1.

4. Use as claimed in any one of Claims 1 to 3, wherein said GI mucosa is selected from intestinal mucosa, small bowel mucosa, large bowel mucosa, the mucosa in the rectum.

5. Use as claimed in Claim 4, wherein said mucosa is the intestinal mucosa.

6. Use as claimed in Claim 4 or 5, wherein said liposomes comprise one or more anionic lipids selected from 1,2-distearoyl-*sn*-glycero-3-[phospho-*rac*-(1-glycerol)] (DSPG), hydrogenated soy phosphatidylglycerol.

7. Use as claimed in any one of Claims 1 to 6, wherein said medicament comprises as an active ingredient an agent effective in inhibiting inflammatory responses.

8. Use according to Claim 7, wherein the active agent is selected from: steroids, salicylates, COX-2 inhibitors, anti-TNFα drugs, antibiotics, immunosupressors, immunomodulators and antioxidants.

9. Use according to Claim 8, wherein the active agent is selected from Prednisone, Prednisolone, methylprednisolone, methylprednisolone succinate, Budesonide, derivatives of 5-aminosalicylic acid, Sulfsalazine, Mesalamine (5ASA), Olsalazine Balsalazide, Metronidazole, Ciprofloxin, Probiotics g. Cyclosporin A, Azathioprine, Methotrexate and 6-Mercaptopurine.

10. Use as claimed in any one of Claims 1 to 6, wherein said medicament comprises as an active ingredient one or more anti-oxidants.

11. Use as claimed in Claim 10, wherein the active agent is selected from tocopherol, free radicals scavengers, SOD and SOD mimics, catalase or therapeutic reducing agents.

## Patentansprüche

1. Verwendung negativ geladener Liposomen, die mit einem nicht kovalent an die Liposomen gebundenen Wirkstoff beladen sind, bei der Herstellung eines Arzneimittels zur Behandlung einer mit der Entzündung der Schleimhaut des gastrointestinalen Trakts (GI-Trakts) assoziierten Krankheit oder Störung, die aus ulzerativer Kolitis, Morbus Crohn und Kolitis ausgewählt ist.

2. Verwendung nach Anspruch 1, worin die Beladung der Liposomen mit dem Wirkstoff die Verkapselung, den Einschluss oder die elektrostatische Adsorption des Wirkstoffs in/an dem Liposom oder jedwede Kombination davon umfasst.

3. Verwendung nach Anspruch 1 oder 2, worin das Arzneimittel zur lokalen Behandlung der nach Anspruch 1 spezifizierten Krankheit oder Störung vorgesehen ist.

4. Verwendung nach einem der Ansprüche 1 bis 3, worin die GI-Schleimhaut aus intestinaler Schleimhaut, Dünndarmschleimhaut, Dickdarmschleimhaut und der Rektumschleimhaut ausgewählt ist.

5. Verwendung nach Anspruch 4, worin die Schleimhaut die intestinale Schleimhaut ist.

6. Verwendung nach Anspruch 4 oder 5, worin die Liposomen ein oder mehr anionische(s) Lipid(e) umfassen, die aus 1,2-Distearoyl-*sn*-glycero-3-[phospho-*rac*-(1-glycerol)] (DSPG) und hydriertem Soja-Phosphatidylglycerol ausgewählt sind.

7. Verwendung nach einem der Ansprüche 1 bis 6, worin das Arzneimittel als Wirkstoff ein Mittel umfasst, das bei der Inhibition von Entzündungsreaktionen wirksam ist.

8. Verwendung nach Anspruch 7, worin der Wirkstoff ausgewählt ist aus: Steroiden, Salicylaten, COX-2-Inhibitoren, Anti-TNFα-Arzneimitteln, Antibiotika, Immunsuppressoren, Immunmodulatoren und Antioxidanzien.

9. Verwendung nach Anspruch 8, worin der Wirkstoff ausgewählt ist aus Prednison, Prednisolon, Methylprednisolon, Methylprednisolon-succinat, Budesonid, Derivaten von 5-Aminosalicylsäure, Sulfsalazin, Mesalamin (5ASA), Olsalazin, Balsalazid, Metronidazol, Ciprofloxin, Probiotika, z. B. Cyclosporin A, Azathioprin, Methotrexat und 6-Mercaptopurin.

10. Verwendung nach einem der Ansprüche 1 bis 6, worin das Arzneimittel ein oder mehr Antioxidans/Antioxidanzien als einen Wirkstoff umfasst.

11. Verwendung nach Anspruch 10, worin der Wirkstoff ausgewählt ist aus Tocopherol, Radikalfängern, SOD und SOD-Mimetika, Katalase oder therapeutischen Reduktionsmitteln.

## Revendications

1. Utilisation de liposomes chargés négativement et chargés d'un ingrédient actif qui n'est pas lié de manière covalente auxdits liposomes, dans la fabrication d'un médicament destiné au traitement d'une maladie ou d'un trouble associé(e) à l'inflammation de la muqueuse du tractus gastro-intestinal (GI), choisi(e) parmi le groupe constitué par la rectocolite hémorragique, la maladie de Crohn et la colite.

2. Utilisation selon la revendication 1, dans laquelle le chargement des liposomes par l'ingrédient actif comprend l'encapsulation, le piégeage ou l'adsorption électrostatique de l'ingrédient actif par le liposome, ou une combinaison quelconque de ceux-ci.

3. Utilisation selon la revendication 1 ou 2, dans laquelle le médicament est destiné au traitement local de la maladie ou du trouble spécifié(e) selon la revendication 1.

4. Utilisation selon l'une quelconque des revendications 1 à 3, dans laquelle ladite muqueuse du GI est choisie parmi le groupe constitué par la muqueuse intestinale, la muqueuse de l'intestin grêle, la muqueuse du gros intestin, la muqueuse du rectum.

5. Utilisation selon la revendication 4, dans laquelle ladite muqueuse est la muqueuse intestinale.

6. Utilisation selon la revendication 4 ou 5, dans laquelle lesdits liposomes comprennent un ou plusieurs lipides anioniques choisis parmi le 1,2-distéaroyl-*sn-*glycéro-3-[phospho-*rac*-(1-glycérol)] (DSPG) et le phosphatidylglycérol de soja hydrogéné.

7. Utilisation selon l'une quelconque des revendications 1 à 6, dans laquelle ledit médicament comprend comme ingrédient actif un agent efficace pour l'inhibition des réponses inflammatoires.

8. Utilisation selon la revendication 7, dans laquelle l'agent actif est choisi parmi: les stéroïdes, les salicylates, les inhibiteurs de COX-2, les médicaments anti-TNFα, les antibiotiques, les immunosuppresseurs, les immunomodulateurs et les antioxydant.

9. Utilisation selon la revendication 8, dans laquelle l'agent actif est choisi parmi le groupe constitué par la Prednisone, la Prednisolone, la méthyl-prednisolone, le succinate de méthyl-prednisolone, le Budésonide, les dérivés de l'acide 5-aminosalicylique, la Sulfsalazine, la Mésalamine (5ASA), l'Olsalazine, le Balsalazide, la Métronidazole, la Ciprofloxine, les Probiotiques par exemple la Ciclosporine A, l'Azathioprine, le Méthotrexate et la 6-Mercaptopurine,

10. Utilisation selon l'une quelconque des revendications 1 à 6, dans laquelle ledit médicament comprend comme ingrédient actif un ou plusieurs antioxydants.

11. Utilisation selon la revendication 10, dans laquelle l'agent actif est choisi parmi le groupe constitué par le tocophérol, les agents anti-radicalaires, la SOD et les mimétiques de SOD, la catalase ou des agents réducteurs thérapeutiques.
